# EUROPEAN PATENT APPLICATION

(11) **EP 1 234 878 A1**
(43) Date of publication of application: **28.08.2002**
(21) Application number: 00977857.2
(22) Date of filing: 22.11.2000
(51) Int. Cl.: C12N 15/12, A61K 31/7105, A61K 48/00, A61P 11/06, G01N 33/50, G01N 33/15

(54) **USE OF DISEASE-ASSOCIATED GENE**

(30) Priority: 24.11.1999 JP 33347999; 27.04.2000 JP 2000127589
(71) Applicant: Takeda Chemical Industries, Ltd., Osaka-shi, Osaka 541-0045 (JP)
(72) Inventor: NAKANISHI, Atsushi, Tsukuba-shi, Ibaraki 305-0821 (JP); MORITA, Shigeru, Tsukuba-shi, Ibaraki 305-0035 (JP)
(74) Representative: Keller, Günter, Dr.
(86) International application number: JP0008232
(87) International publication number: WO01038530

(57) **Abstract**

The present invention provides an antisense DNA to a disease-associated gene, drugs containing the antisense DNA, an antibody to a disease-associated gene product, diagnostics containing the antibody, a method of screening a compound capable of inhibiting the activities of the disease-associated gene product, compounds obtainable by the screening method, etc.

A compound which inhibits the activities of a protein having the same or substantially the same amino acid sequence as the amino acid sequence represented by SEQ ID NO: 1 or its salt and a neutralizing antibody capable of inhibiting the activities of the protein can be used, e.g., for the prevention/treatment of diseases such as bronchial asthma, chronic obstructive pulmonary disease, etc. The antisense DNA can inhibit the expression of a protein having the same or substantially the same amino acid sequence as the amino acid sequence represented by SEQ ID NO: 1, and can be used, e.g., for the prevention/treatment of diseases such as bronchial asthma, chronic obstructive pulmonary disease, etc.

## Description

### FIELD OF THE INVENTION

This invention relates to use of a disease-associated gene. More particularly, the invention relates to an antisense nucleotide to a disease-associated gene, which is useful as a diagnostic marker for bronchial asthma, chronic obstructive pulmonary disease, etc., an antibody against a disease-associated gene product, a method of screening a drug by using the disease-associated gene product, etc.

### BACKGROUND ART

Bronchial asthma is a chronic inflammatory disease of airways showing respiratory stenosis, in which symptoms such as paroxysmal dyspnea, stridor, cough, etc. are observed. Many cells such as bronchial epithelial cells, mast cells, eosinophils, T lymphocytes, etc are involved in its onset and development. One of the most important characteristics of bronchial asthma is that airways are hyperresponsive to irritants (airway hyperresponsiveness). This airway hyperresponsiveness is attributable to airway inflammation caused mainly due to exfoliation of bronchial epithelial cells by neurotransmitters secreted from the cells such as eosinophils, etc., infiltrated into airways, and it is also considered that genetic factors or environmental factors will affect the airway hyperresponsiveness complicatedly.

When the inflammatory reaction of airways is triggered by external irritants (allergens, exhausts) or viral infection, adhesion molecules including VCAM-1, ICAM-1 and the like are expressed on bronchial epithelial cells or on capillary endothelial cells around the bronchi [J. Allergy Clin. Immunol., 96, 941 (1995)] to produce cytokines or chemotactic substances. In patients with bronchial asthma, the function of Th2 type helper T cells is activated to increase the production of Th2 type cytokines such as IL-3, IL-4, IL-5, IL-13, GM-CSF, etc., or chemokines such as eotaxin, RANTES, etc. IL-4 or IL-13 has an activity of inducing IgE production, and IL-3 or IL-4 has an activity of inducing the growth of mast cells. Furthermore, eosinophils differentiate and proliferate in response to IL-5, GM-CSF, etc. and infiltrate into the airways in response to eotaxin or RANTES [Allergy Asthma Proc., 20, 141 (1999)].

Epithelial cells that cover the tracheal and bronchial mucosa not only have the barrier function to prevent direct transmittance of external irritants to submucosal tissues and the function to excrete mucus secretions or foreign matters, but also regulate bronchoconstriction by secreted epithelium-derived smooth muscle relaxing factors, etc. Eosinophils infiltrated into the airways of patient with bronchial asthma release through degranulation intracellular granule proteins such as activated MBP (major basic protein), ECP (eosinophil cationic protein), etc. [Compr. Ther., 20, 651 (1994)]. By the cytotoxic action of these granular proteins, the exfoliation and damages of epithelial cells occur. The exfoliation of epithelial cells leads to exposure of sensory nerve terminals, increase in epithelial permeability and loss of epithelium-derived smooth muscle relaxing factors. Also, leukotriene C4 (LTC4) or platelet activating factor (PAF) produced by eosinophils increase tension of bronchial smooth muscle. It is considered that when the foregoing changes are repeated to make it chronic, the bronchial walls would be thickened to cause airway hyperresponsiveness.

As stated above, it is known that genes of the cytokines or adhesion molecules described above are increasingly expressed, accompanied by inflammation of the airways, but there is no report to systematically analyze the change of a gene, expression of which is localized in the lesion of lung/bronchi and which is associated with the onset of airway hyperresponsiveness.

On the other hand, involvement of chloride channel in a disease is reported only on CFTR associated with cystic fibrosis [Science, 257, 1125 (1992)] and CIC chloride channel with myotonia [Nature, 354, 304 (1991)], but there is no report that calcium-dependent chloride channel would be associated with a disease such as bronchial asthma, etc.

Human-derived CLCA1cDNA and protein and mouse-derived Gob-5cDMA and protein of the present invention are described in the literature [Genomics, 54, 200 (1998) ; Biochem. Biophys. Res. Commun., 255, 347 (1999)], but the literature is silent on any involvement of these DNAs or proteins in bronchial asthma, chronic obstructive pulmonary disease, etc.

The present invention provides a method of screening a compound capable of inhibiting the activity of a protein, expression of which increases in the lung/bronchi having increased airway hyperresponsiveness, a compound obtainable by the screening method, etc.

### DISCLOSURE OF THE INVENTION

The present inventors made extensive investigations to solve the problems above and as a result, discovered a cDNA, which expression markedly increases in the lung/bronchi of mouse asthma model. On further studies of this cDNA expression pattern in detail, it has been revealed that the expression is induced locally in the epithelial cells of airways with allergic airway inflammation.

Based on these findings, the inventors continued further investigations so that the present invention has been attained.

That is, the present invention relates to the following features.
(1) An antisense nucleotide having a base sequence complementary or substantially complementary to a base sequence of a DNA encoding a protein containing the same or substantially the same amino acid sequence as the amino acid sequence represented by SEQ ID NO: 1, or its partial peptide.
(2) The antisense nucleotide according to (1), wherein substantially the same amino acid sequence as the amino acid sequence represented by SEQ ID NO: 1 is the amino acid sequence represented by SEQ ID NO: 2.
(3) The antisense nucleotide according to (1), which has a base sequence complementary to the base sequence represented by SEQ ID NO: 3 or SEQ ID NO: 4, or a part thereof.
(4) A pharmaceutical comprising the antisense nucleotide according to (1).
(5) The pharmaceutical according to (4), which is for the prevention/treatment of bronchial asthma or chronic obstructive pulmonary disease.
(6) A diagnostic comprising an antibody against a protein containing the same or substantially the same amino acid sequence as the amino acid sequence represented by SEQ ID NO: 1, its partial peptide, or a salt thereof.
(7) A method of screening a compound or a salt thereof that inhibits the activity of a protein containing the same or substantially the same amino acid sequence as the amino acid sequence represented by SEQ ID NO: 1, its partial peptide, or a salt thereof, which comprises using a protein containing the same or substantially the same amino acid sequence as the amino acid sequence represented by SEQ ID NO: 1, its partial peptide, or a salt thereof.
(8) The method according to (7), wherein the protein containing the same or substantially the same amino acid sequence as the amino acid sequence represented by SEQ ID NO: 1, its partial peptide, or a salt thereof is the one expressed on cell membrane of the transformant transformed with a DNA containing a DNA encoding the protein containing the same or substantially the same amino acid sequence as the amino acid sequence represented by SEQ ID NO: 1, its partial peptide, or a salt thereof.
(9) A kit for screening a compound or a salt thereof that inhibits the activity of a protein containing the same or substantially the same amino acid sequence as the amino acid sequence represented by SEQ ID NO: 1, its partial peptide, or a salt thereof, comprising a protein containing the same or substantially the same amino acid sequence as the amino acid sequence represented by SEQ ID NO: 1, its partial peptide. or a salt thereof.
(10) A compound or a salt thereof that inhibits the activity of a protein containing the same or substantially the same amino acid sequence as the amino acid sequence represented by SEQ ID NO: 1, its partial peptide, or a salt thereof, which is obtainable using the screening method according to (7) or the screening kit according to (9).
(11) A pharmaceutical comprising a compound or a salt thereof that inhibits the activity of a protein containing the same or substantially the same amino acid sequence as the amino acid sequence represented by SEQ ID NO: 1, its partial peptide, or a salt thereof , which is obtainable using the screening method according to (7) or the screening kit according to (9).
(12) The pharmaceutical according to (11), which is for the prevention/treatment of bronchial asthma or chronic obstructive pulmonary disease.
(13) A preventive/remedy for bronchial asthma or chronic obstructive pulmonary disease, comprising a compound having a calcium-dependent chloride channel inhibiting activity, or a salt thereof.
(14) A method for the prevention/treatment of bronchial asthma or chronic obstructive pulmonary disease, which comprises administering the antisense nucleotide according to (1) to a mammal.
(15) Use of the antisense nucleotide according to (1) for manufacturing a preventive/remedy for bronchial asthma or chronic obstructive pulmonary disease.
(16) A method for the prevention/treatment of bronchial asthma or chronic obstructive pulmonary disease, which comprises administering to a mammal a compound or a salt thereof that inhibits the activity of a protein containing the same or substantially the same amino acid sequence as the amino acid sequence represented by SEQ ID NO: 1,-its partial peptide, or a salt thereof, which is obtainable using the screening method according to (7) or the screening kit according to (9).
(17) Use of a compound or a salt thereof that inhibits the activity of a protein containing the same or substantially the same amino acid sequence as the amino acid sequence represented by SEQ ID NO: 1, its partial-peptide, or a salt thereof, which is obtainable using the screening method according to (7) or the screening kit according to (9), for manufacturing a preventive/remedy for bronchial asthma or chronic obstructive pulmonary disease.
(18) A method for prevention/treatment of bronchial asthma or chronic obstructive pulmonary disease, which comprises administering a compound having a calcium-dependent chloride channel inhibiting activity, or a salt thereof to a mammal.
(19) Use of a compound or its salt having a calcium-dependent chloride channel inhibiting activity, for manufacturing a preventive/remedy for bronchial asthma or chronic obstructive pulmonary disease.

### BRIEF DESCRIPTION OF THE DRAWINGS

FIG. 1 shows tissue distribution of Gob-5 gene (mRNA) in normal mouse and model mouse with increased airway hyperresponsiveness, in which Lu, H, Li, Ki, Br, Thy, Sp, SI, LI, St and PBL designate lung, heart, liver, kidney, brain, thymus, spleen, small intestine, large intestine, stomach and peripheral blood lymphocyte, respectively.
FIG. 2 shows change in increased airway hyperresponsiveness with time course, by administration of acetylcholine(Ach)in a dose of 500 µg/kg. **: p<0.01 (Dunnett's Test)
FIG. 3 shows change with time course in the count of infiltrated cells in the alveolar lavage fluid shown in EXAMPLE 3, wherein Mφ, Eos, Neu and Lym designate macrophage, eosinophil, neutrophil and lymphocyte, respectively.
FIG. 4 shows change of Gob-5 gene (mRNA) with time course in model mouse with increased airway hyperresponsiveness shown in EXAMPLE 3.
FIG. 5 indicates the Gob-5 gene expression site on frozen lung sections from model mouse with increased airway hyperresponsiveness or normal mouse, shown in EXAMPLE 4.
FIG. 6 shows PAS staining of the frozen lung sections from model mouse with increased airway hyperresponsiveness or normal mouse, shown in EXAMPLE 4.
FIG. 7 shows the results of the experiment shown in EXAMPLE 7, in which the antisense nucleotide to Gob-5 gene was administered. **: p<0.01
FIG. 8 shows the airway responsiveness to acetylcholine in mouse administered with AdV Gob-5-AS or control mouse administered with AdV-wt, shown in EXAMPLE 9, wherein the data represent mean values ± standard error. The two groups of the OVA inhalation group and the PBS inhalation group were compared by t test (#: p<0.05, ##: p<0.01) and a multiple comparison between the OVA inhalation group and the AdV inhalation group was made using the Dunnett's test (# : p<0.05, ##: p<0.01).

In the figure, Columns 1, 2, 3 and 4 designate the PBS inhalation group, the OVA inhalation group, the group administered with AdV-wt followed by OVA inhalation, and the group administered with AdV Gob-5-AS followed by OVA inhalation, respectively.

FIG. 9 shows PAS staining of lung frozen sections from mouse administered with AdV Gob-5-AS or control mouse administered with AdV-wt, shown in EXAMPLE 9.

FIG. 10 shows the Cl⁻ ion secretion assay using CHO cells or human CLCA1-expressed CHO cells used in EXAMPLE 10, and changes of fluorescent intensity with time course without stimulation or stimulated with 10 µM of ionomycin.

In the figure, -o-, -●-, -Δ- and -▲- denote CHO cells with no stimulation, CHO cells stimulated with 10 µM of ionomycin, human CLCA1-expressed CHO cells with no stimulation and human CLCA1-expressed CHO cells stimulated with 10 µM of ionomycin, respectively.

FIG. 11 shows the action of human CLCA1-expressed CHO cells shown in EXAMPLE 10 inhibiting the secretion of Cl⁻ ions by NFA (niflumic acid), DIDS (4,4'-diisothiocyanastilbene-2,2'-disulfonic acid) and DTT (dithiothreitol).

In the figure, the columns designate the following cells.
- Column 1:: no stimulation
- Column 2:: added with no anion transportation inhibitor and stimulated with ionomycin (10 µM)
- Column 3:: added with NFA (200 µM) and stimulated with ionomycin (10 µM)
- Column 4:: added with NFA (10 µM) and stimulated with ionomycin (10 µM)
- Column 5:: added with DIDS (200 µM) and stimulated with ionomycin (10 µM)
- Column 6:: added with DIDS (10 µM) and stimulated with ionomycin (10 µM)
- Column 7:: added with DTT (200 µM) and stimulated with ionomycin (10 µM)
- Column 8:: added with DTT (10 µM) and stimulated with ionomycin (10 µM)

### BEST MODE FOR CARRYING OUT THE INVENTION

The protein used in the present invention, which contains the same or substantially the same amino acid sequence as the amino acid sequence represented by SEQ ID NO: 1 (hereinafter sometimes referred to as the protein of the invention or the protein used in the present invention) may be any protein derived from any cells of human and other warm-blooded animals (e.g., guinea pig, rat, mouse, chicken, rabbit, swine, sheep, bovine, monkey, etc.) such as hepatocyte, splenocyte, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocyte or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

As the amino acid sequence having substantially the same amino acid sequence as that shown by SEQ ID NO: 1, there are amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 1.

Preferred examples of the protein containing substantially the same amino acid sequence as the amino acid sequence shown by SEQ ID NO: 1 include proteins having substantially the same amino acid sequence as the amino acid sequence shown by SEQ ID NO: 1 and having a property substantially equivalent to that of the protein having the amino acid sequence shown by SEQ ID NO: 1, etc.

As the substantially equivalent activity, there are, e.g., a chloride channel-like activity (calcium-dependent chloride channel-like activity), and the like. The substantially equivalent is used to mean that the nature of these properties is equivalent in property (e.g., physiologically or pharmacologically). Preferably, the chloride channel-like activity is equivalent (e.g., approximately 0.01 to 100 times, preferably approximately 0.1 to 10 times, more preferably 0.5 to twice), but differences in degree such as a level of these properties, quantitative factors such as a molecular weight, etc. may be present and allowable.

Measurement of the activities such as the chloride channel-like activity, etc. may be made by a modification of publicly known methods; for example, the measurement may be made by the method described in Genomics, 54, 200 (1998) or its modification.

Examples of the protein containing substantially the same amino acid sequence represented by SEQ ID NO: 1 include the protein containing the amino acid sequence represented by SEQ ID NO: 2, and the like.

Furthermore, examples of the protein used in the present invention include so-called muteins such as proteins containing 1) the amino acid sequence represented by SEQ ID NO: 1 or SEQ ID NO: 2, of which at least 1 or 2 (preferably about 1 to about 30, more preferably about 1 to about 10 and most preferably several (1 to 5)) amino acids are deleted; 2) the amino acid sequence represented by SEQ ID NO: 1 or SEQ ID NO: 2, to which at least 1 or 2 (preferably about 1 to about 30, more preferably about 1 to about 10 and most preferably several (1 to 5)) amino acids are added; 3) the amino acid sequence represented by SEQ ID NO: 1 or SEQ ID NO: 2, in which at least 1 or 2 (preferably about 1 to about 30, more preferably about 1 to about 10 and most preferably several (1 to 5)) amino acids are inserted; 4) the amino acid sequence represented by SEQ ID NO: 1 or SEQ ID NO: 2, in which at least 1 or 2 (preferably about 1 to about 30, more preferably about 1 to about 10 and most preferably several (1 to 5)) amino acids are substituted by other amino acids; or 5) a combination of the above amino acid sequences.

When the amino acid sequences are inserted, deleted or substituted as described above, the positions at which the insertion, deletion or substitution occurs are not particularly limited, but may be at the positions other than those of the amino acid sequences common to the amino acid sequence represented by SEQ ID NO: 1 or SEQ ID NO: 2.

Throughout the specification, the proteins are represented in accordance with the conventional way of describing proteins, that is, the N-terminus (amino terminus) at the left hand and the C-terminus (carboxyl terminus) at the right hand. In the proteins used in the present invention including the protein containing the amino acid sequence shown by SEQ ID NO: 1, the C-terminus is usually in the form of a carboxyl group (-COOH) or a carboxylate (-COO⁻) but may be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, it may be amidated or esterified and such an amide or ester is also included within the protein of the present invention. The ester group may be the same group as that described with respect to the above C-terminal.

Furthermore, examples of the protein used in the present invention include variants of the above proteins, wherein the amino group at the N-terminus (e.g., methionine residue) of the protein is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains.

Specific examples of the protein used in the present invention include a mouse-derived protein containing the amino acid sequence represented by SEQ ID NO: 1, a human small intestine-derived protein containing the amino acid sequence represented by SEQ ID NO: 2, and the like.

The partial peptide of the protein used in the present invention may be any peptide, so long as it is a partial peptide of the protein used in the present invention described above and preferably has the property equivalent to that of the protein used in the present invention described above.

Specific examples for the purpose of preparing the antibody of the present invention later described include a peptide having the 22-913 amino acid sequence in the amino acid sequence represented by SEQ ID NO: 1, a peptide having the 22-914 amino acid sequence in the amino acid sequence represented by SEQ ID NO: 2, and the like. Also, for the screening method or the screening kit of the present invention later described, there are preferably employed a peptide containing the cell membrane binding site (the 1-21 amino acid sequence in the amino acid sequence represented by SEQ ID NO: 1 or SEQ ID NO: 2) and having a part of the 22-913 amino acid sequence in the amino acid sequence represented by SEQ ID NO: 1, or having a part of the 22-914 amino acid sequence in the amino acid sequence represented by SEQ ID NO: 2. Preferably used are peptides containing the sequence of at least 20, preferably at least 50, more preferably at least 70, much more preferably at least 100, and most preferably at least 200, amino acids in the constituent amino acid sequence of the protein used in the present invention, and the like.

The partial peptides used in the present invention may be those containing the amino acid sequence, of which at least 1 or 2 (preferably about 1 to about 10 and more preferably several (1 to 5)) amino acids are deleted; to which at least 1 or 2 (preferably about 1 to about 10 and more preferably several (1 to 5)) amino acids are added; in which at least 1 or 2 (preferably about 1 to about 10 and more preferably several (1 to 5)) amino acids are inserted; or, in which at least 1 or 2 (preferably about 1 to about 10 and more preferably several (1 to 5)) amino acids are substituted by other amino acids.

In the partial peptide used in the present invention, the C-terminus is usually in the form of a carboxyl group (-COOH) or a carboxylate (-COO⁻) but may be in the form of an amide (-CONH₂) or an ester (-COOR), as in the aforesaid proteins employed in the present invention.

The partial peptides used in the invention include those containing carboxyl groups (or carboxylates) other than at the C-terminus, those wherein the amino group of the N-terminal amino acid residue (e.g., methionine residue) is protected with a protecting group, those wherein the N-terminal region is cleaved in vivo and the glutamine thus formed is pyroglutaminated, those wherein a substituent on the side chain of an amino acid in the molecule is protected with a suitable protecting group, or conjugated proteins such as so-called glycoproteins to which sugar chains are bound, etc., as in the proteins used in the present invention described above.

The partial peptide used in the invention may also be used as an antigen for producing antibodies.

The protein or its partial peptide used in the present' invention may be employed in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The proteins or partial peptides used in the present invention, or salts thereof, may be manufactured by a publicly known method used to purify a protein from human or other warm-blooded animal cells or tissues described above. Alternatively, they may also be manufactured by culturing transformants containing DNAs encoding the proteins. Furthermore, they may also be manufactured by a modification of the methods for peptide synthesis, which will be later described.

Where these proteins or peptides are manufactured from human or mammalian tissues or cells, human or mammalian tissues or cells are homogenized, then extracted with an acid or the like, and the proteins or peptides are isolated and purified from the extract by a combination of chromatography techniques such as reverse phase chromatography, ion exchange chromatography, and the like.

To synthesize the proteins or partial peptides used in the present invention, or salts thereof or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzylester, 4-methoxybenzylester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower C₁₋₆ alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the desired esterified protein or peptide.

The partial peptide or salts thereof used in the present invention can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in 1) to 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the peptide can be converted into an appropriate salt by a publicly known method; conversely when the partial peptide is obtained in a salt form, it can be converted into a free form or other different salt form by a publicly known method.

The DNA encoding the protein used in the present invention may be any DNA so long as it contains the base sequence encoding the protein used in the present invention described above. The DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Specifically, the DNA encoding the protein used in the present invention may be any one of, for example, a DNA having the base sequence represented by SEQ ID NO: 3 or SEQ ID NO: 4, or any DNA having a base sequence hybridizable to a DNA having the base sequence represented by SEQ ID NO: 3 or SEQ ID NO: 4 under high stringent conditions and encoding a protein which has the properties substantially equivalent to those of the protein used in the present invention.

Specific examples of the DNA that is hybridizable to DNA having the base sequence represented by SEQ ID NO: 3 or SEQ ID NO: 4 under high stringent conditions include DNAs having at least about 50% homology, preferably at least about 60% homology, more preferably at least about 70% homology, much more preferably at least about 80% homology, further much more preferably at least about 90% homology and most preferably at least about 95% homology, to the base sequence represented by SEQ ID NO: 3 or SEQ ID NO: 4.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration at about 19 mM at a temperature of about 65°C are most preferred.

More specifically, as the DNA encoding the protein having the amino acid sequence represented by SEQ ID NO: 1, there may be employed a DNA containing a DNA having the base sequence represented by SEQ ID NO: 3. As the DNA encoding the protein having the amino acid sequence represented by SEQ ID NO: 2, there may be employed a DNA containing a DNA having the base sequence represented by SEQ ID NO: 4.

The DNA encoding the partial peptide used in the present invention may be any DNA so long as it contains the base sequence encoding the partial peptide used in the present invention described above. The DNA may also be any of genomic DNA, genomic DNA library, cDNA derived from the cells and tissues described above, cDNA library derived from the cells and tissues described above and synthetic DNA.

Specifically as the DNA encoding the partial peptide used in the present invention, there may be employed any one of a DNA having a part of a DNA containing the base sequence represented by SEQ ID NO: 3 or SEQ ID NO: 4, a DNA having a base sequence hybridizable to a DNA having the base sequence represented by SEQ ID NO: 3 or SEQ ID NO: 4 under high stringent conditions and containing a part of a DNA encoding a protein having an activity substantially equivalent to that of the protein used in the present invention, and the like.

The DNA hybridizable to the base sequence represented by SEQ ID NO: 3 or SEQ ID NO: 4 has the same significance as described hereinabove.

Methods for the hybridization and the high stringent conditions that can be used are the same as those described above.

As a means of cloning a DNA that completely encodes the protein or partial peptide used in the present invention (hereinafter sometimes merely referred to as the protein of the present invention in describing the cloning and expression of the DNAs encoding the same), the DNA may be amplified either by PCR using synthetic DNA primers containing a part of the base sequence of the protein of the present invention, or the DNA inserted into an appropriate vector may be screened by hybridization with a labeled DNA fragment or synthetic DNA that encodes a part or entire region of the protein of the present invention. The hybridization can be carried out. for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method or the Kunkel method, or modifications thereof by using publicly known kits available as Mutan™-super Express Km, Mutan™-K (both manufactured by Takara Shuzo Co., Ltd.), etc.

The cloned DNA encoding the protein can be used as it is depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the protein of the present invention can be manufactured, for example, by (a) excising the desired DNA fragment from the DNA encoding the protein of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pAl-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, γPL promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultured generally at about 30°C to about 40°C for about 6 hours to about 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the protein of the present invention can be produced intracellularly, on the cell membrane, or extracellularly of the transformant.

The protein of the present invention can be separated and purified from the culture described above by the following procedures.

When the protein of the present invention is extracted from the culture or cells, the transformant or cell is collected after culturing by a publicly known method and suspended in a appropriate buffer. The transformant or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the transformant or cell to collect the supernatant by a publicly known method.

The supernatant or the protein contained in the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.

The presence of the thus produced protein of the present invention can be determined by a binding test to a labeled ligand and by an enzyme immunoassay using a specific antibody.

The antibodies to the protein or partial peptide, or its salts used in the present invention may be any of polyclonal and monoclonal antibodies, as long as they are capable of recognizing the protein or partial peptide, or its salts, used in the present invention.

The antibodies to the protein or partial peptide, or its salts used in the present invention (hereinafter sometimes simply referred to as the protein of the invention in describing the antibodies) may be produced by a publicly known method of producing an antibody or antiserum, using the protein of the-invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and twice to ten times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be effected in a medium for animal cells supplemented with HAT (hypoxanthine, aminopterin and thymidine) . Any screening and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20°C to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense nucleotide having a complementary or substantial complementary base sequence to the DNA encoding the protein or partial peptide used in the present invention (hereinafter these DNAs are sometimes collectively referred to as the DNAs of the present invention in the following description of antisense nucleotide) can be any antisense nucleotide, so long as it possesses a base sequence complementary or substantially complementary to the base sequence of the DNA of the present invention and capable of suppressing expression of the DNA, but it is preferably an antisense DNA.

The base sequence substantially complementary to the DNA of the present invention may, for example, be a base sequence having at least about 70% homology, preferably at least about 80% homology, more preferably at least-about 90% homology and most preferably at least about 95% homology, to the full-length base sequence or partial base sequence of the base sequence complementary to the DNA of the present invention (i.e., complementary strand to the DNA used in the present invention), and the like. In the entire base sequence of the complementary strand to the DNA of the present invention, an antisense nucleotide having at least about 70% homology, preferably at least about 80% homology, more preferably at least about 90% homology and most preferably at least about 95% homology, to the complementary strand of the base sequence which encodes the N-terminal region in the protein of the present invention (e.g., the base sequence around the initiation codon).

Specific examples include an antisense nucleotide containing a base sequence complementary or substantially complementary to the base sequence of a DNA having the base sequence represented by SEQ ID NO: 3, SEQ ID NO: 4, SEQ ID NO: 22 or SEQ ID NO: 26, or a part of the complementary or substantially complementary base sequence (more preferably an antisense nucleotide containing a base sequence complementary to the base sequence of a DNA having the base sequence represented by SEQ ID NO: 3 or SEQ ID NO: 4, or a part thereof); an antisense nucleotide containing a base sequence complementary to the base sequence of a DNA having the 1-14 base sequence represented by SEQ ID NO: 22, or a part thereof; an antisense nucleotide containing a base sequence complementary to the base sequence of a DNA having the 2764-2843 base sequence represented by SEQ ID NO: 22, or a part thereof; an antisense nucleotide containing a base sequence complementary to the base sequence of a DNA having the 1-22 base sequence represented by SEQ ID NO: 26, or a part thereof; an antisense nucleotide containing a base sequence complementary to the base sequence of a DNA having the 2765-2825 base sequence represented by SEQ ID NO: 26, or a part thereof; and the like.

The antisense nucleotide is generally constructed by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methylphosphonate, phosphorodithionate, etc. These anti sense nucleotides may be synthesized using a publicly known DNA synthesizer, etc.

Hereinafter, the protein or partial peptide, or its salts of the present invention (hereinafter sometimes merely referred to as the protein of the present invention), the DNA encoding the protein or partial peptide (hereinafter sometimes merely referred to as the DNA of the present invention), the antibody to the protein, partial peptide, or its salts of the present invention (hereinafter sometimes merely referred to as the antibody of the present invention) and the antisense nucleotide to the DNA of the present invention (hereinafter sometimes merely referred to as the anti sense nucleotide of the present invention) are explained with respect to the utilities.

The protein of the present invention can be utilized as a disease marker, since expression of the protein increases tissue-specifically in the lung/bronchi of asthma model animal. That is, the protein is useful as a marker for early diagnosis in lung/chest diseases accompanied by inflammation of the lung/airways, judgment of severity in conditions, or predicted development of diseases.

Furthermore, as will be described in EXAMPLES below, increased airway hyperresponsiveness was suppressed by administering the antisense nucleotide of gene encoding the protein of the present invention. It is thus considered that a compound or its salt that inhibits the activities of the protein of the present invention, or the antibody against the protein of the present invention would exhibit the same activity.

Therefore, pharmaceuticals containing the antisense nucleotide of the gene encoding the protein of the present invention, the compound or its salt that inhibits the activities of the protein of the present invention, or the antibody against the protein of the present invention, may be employed as therapeutic/preventive agents for the lung/chest diseases accompanied by inflammation of lung or airways, such as bronchial asthma, chronic obstructive pulmonary disease, etc.

### (1) Screening of drug candidate compounds for disease

Since the protein of the present invention is increasingly expressed prior to inflammation of the lung/bronchi, the compound or its salt that inhibits the activities of the protein of the present invention can be used as a pharmaceutical for the treatment/prevention of lung/chest diseases accompanied by inflammation of the lung/airways, including bronchial asthma, chronic obstructive pulmonary disease, etc.

Therefore, the protein of the present invention is useful as a reagent for screening the compound or its salts that inhibit the activities of the protein of the present invention.

That is, the present invention provides:
(1) a method for screening the compound or its salts that inhibit the activities (e.g., a chloride channel activity, etc.) of the protein of the present invention (hereinafter sometimes merely referred to as the inhibitor), which comprises using the protein of the present invention. More specifically, the present invention provides, e.g.,
(2) a method for screening the inhibitor, which comprises comparing (i) the case where a cell capable of producing the protein of the present invention is activated with a calcium activator and (ii) the case where a mixture of a cell capable of producing the protein of the present invention and a test compound is activated with a calcium activator.

Specifically, in the screening method described above, the method is characterized by measuring, e.g., the chloride channel activity of the protein of the present invention in the cases (i) and (ii), and comparing them.

As the calcium activator, there are employed, e.g., ionomycin, A23187 (calcimycin), and the like.

Examples of the test compound included are a peptide, a protein, a non-peptide compound, a synthetic compound, a fermentation product, a cell extract, a plant extract, an animal tissue extract, and the like. These compounds may be novel compounds or publicly known compounds.

To perform the screening method described above, the protein of the present invention is suspended in a buffer suitable for screening to prepare a specimen for the protein of the present invention. Any buffer having pH of approximately 4 to 10 (desirably a pH of approximately 6 to 8) such as a phosphate buffer, Tris-hydrochloride buffer, etc. maybe used, so long as it does not interfere the chloride channel activity of the protein of the present invention.

As the cell capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, on the cell membrane of which the protein of the present invention has been expressed, e.g., by culturing through the procedure described above, is preferably employed.

The chloride channel activity of the protein of the present invention can be assayed by the method described in, e.g., Genomics, 54, 200 (1998), or its modification.

For example, when a test compound inhibits the chloride channel activity in the case (ii) described above by at least about 20%, preferably at least 30%, more preferably at least about 50%, as compared to the case (i) above, the test compound can be selected to be a compound capable of inhibiting the activity of the protein of the present invention.

The screening kit of the present invention comprises the protein, partial peptide or its salts used in the present invention, or the cell capable of producing the protein or partial peptide used in the present invention.

The compounds or salts thereof obtained using the screening method or screening kit of the present invention are compounds selected from the test compounds described above, for example, peptides, proteins, non-peptide compounds, synthetic compounds, fermentation products, cell extracts, plant extracts, animal tissue extracts, blood plasma, etc., and are the compounds or salts thereof that inhibit the activities (e.g., a chloride channel activity, etc.) of the protein of the present invention.

As salts of these compounds, there may be employed the same salts as those of the protein of the present invention described above.

The compound or its salt that inhibits the activities of the protein of the present invention is useful as a pharmaceutical for the treatment/prevention of lung/chest diseases accompanied by inflammation of the lung/airways, including bronchial asthma, chronic obstructive pulmonary disease, etc.

When the compounds or salts thereof obtained using the screening methods or screening kits of the present invention are used as the therapeutic/prophylactic agents described above, these compounds may be prepared into pharmaceutical preparations in a conventional manner. The compounds may be prepared, for example, in the form of tablets, capsules, elixirs, microcapsules, a sterile solution, a suspension, etc.

Since the thus obtained pharmaceutical preparation is safe and low toxic, the preparation can be administered orally or non-orally to human or other warm-blooded animal (e.g., mouse, rat, rabbit, sheep, swine, bovine, horse, chicken, cat, dog, monkey, chimpanzee, etc.).

The dose of the compound or salt thereof varies depending on its action, target disease, subject to be administered, route for administration, etc.; when the compound or its salt that inhibits the activities of the protein of the present invention is orally administered for the treatment of, e.g., bronchial asthma, the compound or its salt is normally administered in a dose of about 0.1 to about 100 mg, preferably about 1.0 to about 50 mg, and more preferably about 1.0 to about 20 mg per day for adult (as 60 kg body weight) . In parenteral administration, a single dose of the compound or its salt varies depending on subject to be administered, target disease, etc. but it is advantageous for the treatment of, e.g., bronchial asthma, to administer the compound or its salt capable of inhibiting the activities of the protein of the present invention intravenously in the form of injection in a daily dose of about 0.01 to about 30mg, preferably about 0.1 to about 20 mg, and more preferably about 0.1 to about 10 mg for adult (as 60 kg body weight). For other animal species, the corresponding dose as converted per 60 kg body weight can be administered.

### (2) Quantification for the protein of the invention or its peptide, or salts thereof

The antibody to the protein of the present invention (hereinafter sometimes merely referred to as the antibody of the present invention) is capable of specifically recognizing the protein of the present invention, and can thus be used for a quantification of the protein of the present invention in a test sample fluid, in particular, for a quantification by sandwich immunoassay, etc.

That is, the present invention provides:
(i) a method for quantification of the protein of the present invention in a test sample fluid, which comprises competitively reacting the antibody of the present invention, a test sample fluid and the labeled protein of the present invention, and measuring the ratio of the labeled protein of the present invention bound to said antibody; and,
(ii) a method for quantification of the protein of the present invention in a test sample fluid, which comprises reacting the test sample fluid simultaneously or continuously with the antibody of the present invention immobilized on a carrier and another labeled antibody of the present invention, and then measuring the activity of the labeling agent on the immobilized carrier.

In the method (ii) for quantification described above, it is preferred that one antibody is capable of recognizing the N-terminal region of the protein of the present invention, while another antibody is capable of recognizing the C-terminal region of the protein of the present invention.

The monoclonal antibody to the protein of the present invention (hereinafter sometimes referred to as the monoclonal antibody of the invention) may be used to assay the protein of the present invention. Furthermore, the protein can be detected by means of a tissue staining;as well. For these purposes, the antibody molecule per se may be used or F(ab')₂, Fab' or Fab fractions of the antibody molecule may also be used.

There is no particular limitation to the method for quantification of the protein of the present invention using the antibody of the present invention; any method may be used, so far as it relates to a method, in which the amount of an antibody, antigen or antibody-antigen complex can be detected by a chemical or physical means, depending on or corresponding to the amount of antigen (e.g., the amount of a protein) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.

Examples of the labeling agent used in the assay method using the labeling substance are radioisotopes, enzymes, fluorescent substances, luminescent substances, and the like. Examples of the radioisotope are [¹²⁵I], [¹³¹I], [³H], [¹⁴C], etc. Preferred examples of the enzyme described above are those that are stable and have a high specific activity, which include β-galactosidase, β-glucosidase, alkaline phosphatase, peroxidase, malate dehydrogenase, etc. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may also be used for binding of an antibody or antigen to a labeling agent.

In the immobilization of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene. polyacrylamide and silicone; glass; etc.

In the sandwich method, a test sample fluid is reacted with an immobilized monoclonal antibody of the present invention (first reaction), then reacted with another labeled monoclonal antibody of the present invention (second reaction) and the activity of the labeling agent on the immobilized carrier is assayed, whereby the amount of the protein of the present invention in the test sample fluid can be quantified. The first and second reactions may be carried out in a reversed order, simultaneously or sequentiallywith an interval. The type of the labeling agent and the method for immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method according to the present invention for assaying the protein of the present invention by the sandwich method, preferred monoclonal antibodies of the present invention used for the first and the second reactions are antibodies, which binding sites to the protein of the present invention are different from one another. That is, the antibodies used in the first and the second reactions are those wherein, when the antibody used in the second reaction recognizes the C-terminal region of the protein of the present invention, the antibody recognizing the site other than the C-terminal regions, e.g., recognizing the N-terminal region, is preferably used in the first reaction.

The monoclonal antibody of the present invention may be used in an assay system other than the sandwich method, such as a competitive method, an immunometric method, a nephrometry, etc.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, a laser nephrometry utilizing laser scattering can be suitably used.

In applying each of those immunoassays to the assay method of the present invention, any special conditions or operations are not required to set forth. The assay system for the protein of the present invention may be constructed in addition to conditions or operations conventionally used for each of the methods, taking the technical consideration of one skilled in the art into account consideration. For the details of such conventional technical means, a variety of reviews, reference books, etc. may be referred to.

For example, there are Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.)

As described above, the protein of the present invention can be quantified with high sensitivity, using the antibody of the present invention.

Furthermore, (1) when an increased level of the protein of the present invention is detected by quantifying the level of the protein of the present invention using the antibody of the present invention, it can be diagnosed that one suffers from diseases such as lung/chest diseases accompanied by inflammation of the lung/airways, including bronchial asthma, chronic obstructive pulmonary disease, etc., or it is highly likely to suffer from these disease in the future.

The antibody of the present invention can be employed for detecting the protein of the present invention, which is present in a test sample fluid such as a body fluid, a tissue, etc. The antibody can also be used to prepare an antibody column for purification of the protein of-the present invention, detect the protein of the present invention in each fraction upon purification, and analyze the behavior of the protein of the present invention in the cells under investigation.

### (3) Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, chicken, sheep, swine, bovine, horse, cat, dog, monkey, chimpanzee, etc.)can be detected. Therefore, the DNA is useful as a gene diagnostic agent for detecting damages to the DNA or mRNA, its mutation, or decreased expression, increased expression, overexpression, etc. of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

In case that overexpression is detected by, e.g., the Northern hybridization or DNA mutation is detected by the PCR-SSCP assay, it can be diagnosed that it is highly likely to suffer from diseases such as lung/chest diseases accompanied by inflammation of the lung/airways including bronchial asthma, chronic obstructive pulmonary disease, etc.

### (4) Pharmaceutical comprising an antisense nucleotide

The antisense nucleotide of the present invention that binds complementarily to the DNA of the present invention to inhibit expression of the DNA can be used as the agent for the treatment/prevention of diseases such as lung/chest diseases accompanied by inflammation of the lung/airways including bronchial asthma, chronic obstructive pulmonary disease, etc., since the antisense nucleotide is low toxic and can suppress the function (e.g., a chloride channel activity) of the protein of the present invention or the DNA of the present invention in vivo.

In the case that the antisense nucleotide described above is used as the therapeutic/prophylactic agent described above, the antisense nucleotide can be prepared into pharmaceutical preparations and administered by a publicly known method.

For example, when the antisense nucleotide is used, the antisense nucleotide is administered directly, or the antisense nucleotide is inserted into an appropriate vector such as retrovirus vector, adenovirus vector, adenovirus-associated virus vector, etc., followed by treating in a conventional manner. The antisense nucleotide may be administered as it stands, or with a physiologically acceptable carrier to assist its uptake by gene gun or through a catheter such as a catheter with a hydrogel. Alternatively, the antisense nucleotide may be prepared into an aerosol, which is locally administered intrabronchially as an inhalant.

The dose of the antisense nucleotide varies depending on target disease, subject to be administered, route for administration, etc.; for example, when the antisense nucleotide of the present invention is locally administered intrabronchially as an inhalant for the treatment of, e.g., bronchial asthma, the dose is normally about 0.1 to about 100 mg per day for adult (as 60 kg body weight).

In addition, the antisense nucleotide may also be employed as an oligonucleotide probe for diagnosis to examine the presence of the DNA of the present invention in tissues or cells and states of its expression.

### (5) Pharmaceutical comprising the antibody of the present invention

The antibody of the present invention having an activity of neutralizing the protein of the present invention can be used as a medicament for diseases such as lung/chest diseases accompanied by inflammation of the lung/airways including bronchial asthma, chronic obstructive pulmonary disease, etc.

The aforesaid therapeutic/prophylactic agent containing the antibody of the invention for the diseases described above is low toxic and can be administered orally or parenterally to human or other warm-blooded animal (e.g., rat, rabbit, sheep, swine, bovine, cat, dog, monkey, etc.), in its liquid form as it stands, or as a pharmaceutical composition in a suitable preparation form. The dose varies depending on subject to be administered, target disease, condition, route for administration, etc.; when the pharmaceutical is administered to adult for the treatment/prevention of, e.g., bronchial asthma, the antibody of the present invention is normally advantageously administered intravenously, about 1 to about 5 times a day, preferably about 1 to 3 times a day, in a single dose of about 0.01 to about 20 mg/kg body weight, preferably about 0.1 to about 10 mg/kg body weight, and more preferably about 0.1 to about 5 mg/kg body weight for adult. For other parenteral administration and oral administration, the dose corresponding to the dose above can be administered; when the condition is especially severe, the dose may be increased accordingly to the condition.

The antibody of the present invention may be administered in itself or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.

That is, examples of the composition for oral administration include solid or liquid preparations; specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular and drip injections, etc. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, alcohol (e.g., ethanol), polyalcohol (e.g., propylene glycol, polyethylene glycol), nonionic surfactant (e.g., polysorbate 80™, HCO-50 (polyoxyethylene (50 mol) adduct of hydrogenated castor oil)) may be used in combination. For the oily solution, for example, sesame oil, soybean oil and the like are used, and dissolution aids such as benzyl benzoate and benzyl alcohol may be used in combination. The thus-prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of 5 to 500 mg per unit dosage form, 5 to 100 mg especially for injections and 10 to 250 mg for other dosage forms.

Each composition described above may further contain other active components unless formulation with the antibody causes any adverse interaction.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid

Substituents, protecting groups, and reagents used in this specification are presented as the codes below.
- Me :: methyl group
- Et :: ethyl group
- Bu :: butyl group
- Ph :: phenyl group
- TC :: thiazolidine-4(R)-carboxamide group
- Tos :: p-toluenesulfonyl
- CHO :: formyl
- Bzl :: benzyl
- Cl₂-Bzl :: 2,6-dichlorobenzyl
- Bom :: benzyloxymethyl
- Z :: benzyloxycarbonyl
- Cl-Z :: 2-chlorobenzyl oxycarbonyl
- Br-Z :: 2-bromobenzyl oxycarbonyl
- Boc :: t-butoxycarbonyl
- DNP :: dinitrophenol
- Trt :: trityl
- Bum :: t-butoxymethyl
- Fmoc :: N-9-fluorenyl methoxycarbonyl
- HOBt :: 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC :: N,N'-dichlorohexylcarbodiimide

The sequence identification numbers in the sequence listing of the specification indicate the following sequences.

### [SEQ ID NO: 1]

This shows the amino acid sequence of mouse Gob-5 protein.

### [SEQ ID NO: 2]

This shows the amino acid sequence of human CLCA1 protein.

### [SEQ ID NO: 3]

This shows the base sequence of the DNA encoding mouse Gob-5 protein having the amino acid sequence represented by SEQ ID NO: 1.

### [SEQ ID NO: 4]

This shows the base sequence of the DNA encoding the human CLCA1 protein having the amino acid sequence represented by SEQ ID NO: 2.

### [SEQ ID NO: 5]

This shows the base sequence of primer PR1 used in EXAMPLE 1.

### [SEQ ID NO: 6]

This shows the base sequence of primer PR2 used in EXAMPLE 1.

### [SEQ ID NO: 7]

This shows the base sequence of primer PR3 used in EXAMPLE 1.

### [SEQ ID NO: 8]

This shows the base sequence of primer PR4 used in EXAMPLE 1.

### [SEQ ID NO: 9]

This shows the base sequence of primer PR5 used in EXAMPLE 1.

### [SEQ ID NO: 10]

This shows the base sequence of primer PR6 used in EXAMPLE 1.

### [SEQ ID NO: 11]

This shows the base sequence of primer PR7 used in EXAMPLE 1.

### [SEQ ID NO: 12]

This shows the base sequence of primer PR8 used in EXAMPLE 1.

### [SEQ ID NO: 13]

This shows the base sequence of primer PR9 used in EXAMPLE 1.

### [SEQ ID NO: 14]

This shows the base sequence of primer PR10 used in EXAMPLE 1.

### [SEQ ID NO: 15]

This shows the base sequence of primer PR11 used in EXAMPLE 1.

### [SEQ ID NO: 16]

This shows the base sequence of primer PR12 used in EXAMPLE 1.

### [SEQ ID NO: 17]

This shows the base sequence of primer PR13 used in EXAMPLE 1.

### [SEQ ID NO: 18]

This shows the base sequence of primer PR14 used in EXAMPLE 1.

### [SEQ ID NO: 19]

This shows the base sequence of primer PR15 used in EXAMPLE 1.

### [SEQ ID NO: 20]

This shows the base sequence of primer PR16 used in EXAMPLE 1.

### [SEQ ID NO: 21]

This shows the base sequence of primer PR17 used in EXAMPLE 1.

### [SEQ ID NO: 22]

This shows the base sequence of cDNA containing the full-length gene of Gob-5 acquired in EXAMPLE 1.

### [SEQ ID NO: 23]

This shows the sequence of Gob-5 gene probe used in EXAMPLE 2.

### [SEQ ID NO: 24]

This shows the base sequence of primer PR18 used in EXAMPLE 5.

### [SEQ ID NO: 25]

This shows the base sequence of primer PR19 used in EXAMPLE 5.

### [SEQ ID NO: 26]

This shows the base sequence of cDNA containing the full-length gene of CLCA1 acquired in EXAMPLE 5.

### [SEQ ID NO: 27]

This shows the base sequence of primer PR20 used in EXAMPLE 5.

### [SEQ ID NO: 28]

This shows the base sequence of primer PR21 used in EXAMPLE 5.

### [SEQ ID NO: 29]

This shows the base sequence of primer PR22 used in EXAMPLE 5.

### [SEQ ID NO: 30]

This shows the base sequence of primer PR23 used in EXAMPLE 5.

### [SEQ ID NO: 31]

This shows the base sequence of primer PR24 used in EXAMPLE 5.

### [SEQ ID NO: 32]

This shows the base sequence of primer PR25 used in EXAMPLE 5.

### [SEQ ID NO: 33]

This shows the base sequence of primer PR26 used in EXAMPLE 5.

### [SEQ ID NO: 34]

This shows the base sequence of primer PR27 used in EXAMPLE 5.

### [SEQ ID NO: 35]

This shows the base sequence of primer PR28 used in EXAMPLE 5.

### [SEQ ID NO: 36]

This shows the base sequence of primer PR29 used in EXAMPLE 5.

### [SEQ ID NO: 37]

This shows the base sequence of primer PR30 used in EXAMPLE 5.

### [SEQ ID NO: 38]

This shows the base sequence of primer PR31 used in EXAMPLE 5.

### [SEQ ID NO: 39]

This shows the base sequence of oligonucleotide Gob-5AS used in EXAMPLE 7.

### [SEQ ID NO: 40]

This shows the base sequence of oligonucleotide Gob-5MM used in EXAMPLE 7.

### EXAMPLES

Hereinafter the present invention will be described in more detail with reference to EXAMPLES but is not deemed to be limited thereto. The gene manipulation procedures using *Escherichia coli* were performed according to the methods described in the Molecular Cloning.

*Escherichia coli* JM109/pT7-mGob-5 obtained in EXAMPLE 1 by cloning the Gob-5 full length gene DNA-fragment to pT7 Blue-T Vector was on deposit with the Ministry of International Trade and Industry, Agency of Industrial Science and Technology, National Institute of Bioscience and Human Technology (NIBH) at 1-3, Higashi 1-chome, Tsukuba-shi,Ibaraki-ken, Japan (zip code 305-8566), as the Accession Number FERM BP-6882 on September 20, 1999 and with Institute for Fermentation, Osaka (IFO) at 17-85, Juso-honmachi 2-chome, Yodogawa-ku, Osaka-shi, Japan (zip code 532-8686), as the Accession Number IFO 16316 on August 24, 1999.

*Escherichia coli* DH5α/pcDNA-hCLCA1 transformed by cloning with plasmid pcDNA-hCLCA1 obtained in EXAMPLE 6 was on deposit with NIBH as the Accession Number FERM BP-6877 on September 20, 1999 and with IFO as the Accession Number IFO 16311 on August 24, 1999.

### EXAMPLE 1

### Cloning of Gob-5 gene showing increased expression in model mouse with increased airway hyperresponsiveness

Model mice with increased airway hyperresponsiveness were prepared through sensitization by intraperitoneally injecting 400 µl of saline containing 20 µg of OVA (ovalbumin) and 2 mg of alum to BALB/c mice (male, 6 weeks old) and then boosting by intraperitoneal injection of 200 µl of saline containing 10 µg of OVA and 1 mg of alum to the animals one week after, followed by inhalation of 5% OVA solution dissolved in PBS of 1/2 concentration for 25 minutes under unanaesthetised and spontaneous respiration conditions over 7 consecutive days from one week after. Aerozollization was carried out using a ultrasonic nebulizer (Soniclizer 305, ATOM Medical). Increased airway hyperresponsiveness was determined by the Konzett-Rossler method in terms of bronchoconstriction induced by acetylcholine (62.5 - 2000 µg/kg) given 24 hours after the final antigen inhalation. Bronchoalveolar lavage fluid (BALF) was prepared, after death under pentobarbital anesthesia, by inserting a tracheal cannula into the animal and washing 3 times with 0.5 ml of PBS. Next, a smear specimen was prepared using cytospin (700 rpm, 1 min.). After Diff-Quick staining and microscopic inspection, the proportion of macrophages, eosinophils, neutrophils, lymphocytes and other cells was calculated.

Poly (A) +RNA used as a sample was prepared by extracting the total RNA from the lung/bronchi of normal mice and the lung/bronchi of model mice with increased airway hyperresponsiveness, using ISOGEN (manufactured by Wako Pure Chemical Industries, Ltd.), and then passing through oligo-dT cellulose column (manufactured by Pharmacia). Using 2 µg aliquot each of these poly (A) +RNAs as the starting material, cDNA fragments (fragments wherein a part of cDNA is amplified by PCR) specifically expressed in the lung/bronchi of model mice with increased airway hyperresponsiveness were collected by subtraction using PCR-select cDNA subtraction kit (manufactured by Clontech Laboratories, Inc.). The adaptor sequence for the subtraction added to the resulting PCR fragment at the both ends thereof was removed by digestion with restriction enzyme RsaI to change to the DNA fragment with blunt ends. The fragment was then subcloned to pT7Blue T-Vector (manufactured by Novagen, Inc.). The DNA base sequence of the subcloned cDNA fragment was decoded, and based on the decoded base sequence, homology search was conducted by blast N using public Geneble database.

The result revealed that 79 out of 120 clones checked on all coincided with the base sequence encoding the known mouse Gob-5 gene. Thus, cDNA was synthesized from poly (A) +RNA in the lung of model mouse with increased airway hyperresponsiveness, using cDNA synthesis kit (manufactured by Takara Shuzo Co., Ltd.). This cDNA was used as a template and PCR was carried out using 2 primers of 5'-non-translational region (PR1: SEQ ID NO: 5) and 3,-non-translational region (PR2: SEQ ID NO: 6) of the Gob-5 gene to acquire the Gob-5 full-length gene (SEQ ID NO: 22). Using Takara EX Taq (manufactured by Takara Shuzo Co., Ltd.), the reaction was carried out by initially reacting at 98°C for 1 minute and then repeating 30 cycles in Thermal Cycler Gene Amp PCR System 9700 (manufacturedbyPerkin-Elmer, Inc.), in which one cycle was set to include 98°C for 10 seconds, 60°C for 1 minute and then 72°C for 3 minutes, and finally by reacting at 72°C for 10 minutes. The resulting Gob-5 full-length gene DNA fragment was cloned to pT7 Blue-T Vector. Using synthetic primers (PR1 to PR17: SEQ ID NO: 5 to SEQ ID NO: 21), cycle sequencing was conducted to identify the base sequence of the product obtained by a fluorescent DNA sequencer (ABI PRISM TM377, manufactured by Perkin-Elmer, Inc.).

### EXAMPLE 2

### Analysis on tissue distribution of the Gob-5 gene in model mice with increased airway hyperresponsiveness

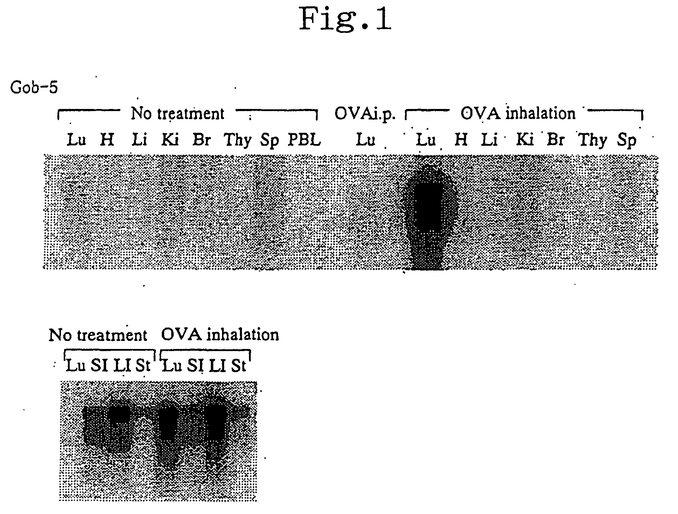

Each organ (lung, heart, liver, kidney, brain, thymus, spleen, small intestine, large intestine, stomach) was isolated from normal mice and model mice with increased airway hyperresponsiveness, and total RNAs were prepared therefrom, using ISOGEN (manufactured by Wako Pure Chemical Industries, Ltd.). The total RNAs were passed through oligo-dT cellulose column (manufactured by Pharmacia, Inc.) to prepare poly (A) +RNAs. After 0.5 µg of this poly (A) +RNAs were electrophoresed on 1.1% agarose gel electrophoresis containing 2.2 M formalin, the RNAs were blotted to nylon membrane filters (Hybond-N+, made by Amersham Pharmacia Biotech, Inc.) by capillary blotting for 18 hours. The RNAs were fixed on the nylon membrane filters through UV treatment, followed by prehybridization at 65°C in Express Hyb Hybridization Solution (manufactured by Clontech Laboratories, Inc.). On the other hand, one (SEQ ID NO: 23) of the Gob-5 cDNA fragments shown in EXAMPLE 1 was labeled with [α-³²P]dCTP using Bca BEST Labeling Kit (manufactured by Takara Shuzo Co., Ltd.) as a probe. Hybridization was carried out at 65°C for 2 hours in Express Hyb Hybridization Solution. Filters were finally rinsed with 0.1 x SSC in 0.1% SDS solution at 50°C followed by detection using BAS-2000 (manufactured by Fuji Photo Film Co., Ltd.). As a result, expression of the Gob-5 gene (mRNA) was observed in the small intestine, large intestine and stomach of normal mouse but not in the lung. In the mouse with increased airway hyperresponsiveness, the expression was markedly observed in the lung, indicating that the expression of Gob-5 was strongly induced accompanied by increased airway hyperresponsiveness. An increase of the Gob-5 expression in the small intestine, large intestine and stomach in the model mouse with increased airway hyperresponsiveness was on the same level as observed in the normal mouse. No potentiation of expression accompanied by increased airway hyperresponsiveness was noted (FIG. 1).

### EXAMPLE 3

### Analysis on the Gob-5 gene with passage of time in model mouse with increased airway hyperresponsiveness

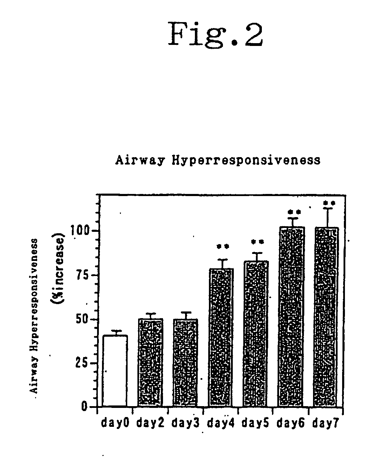

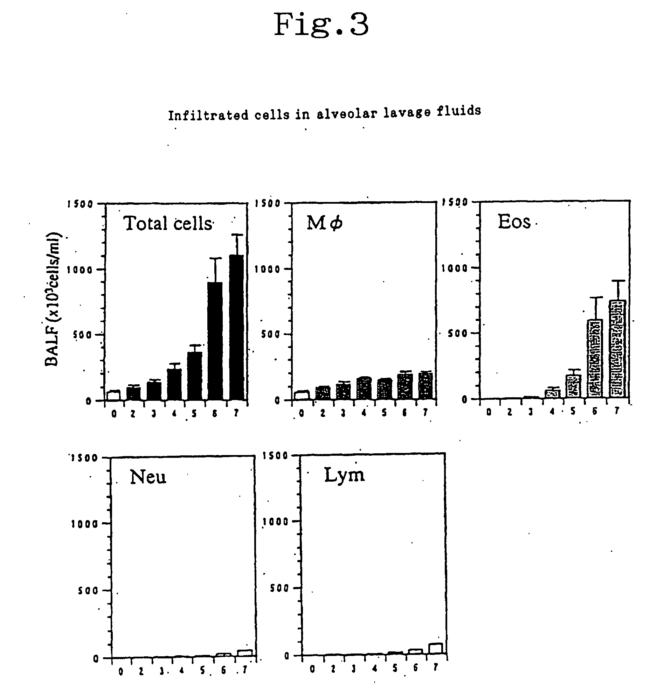

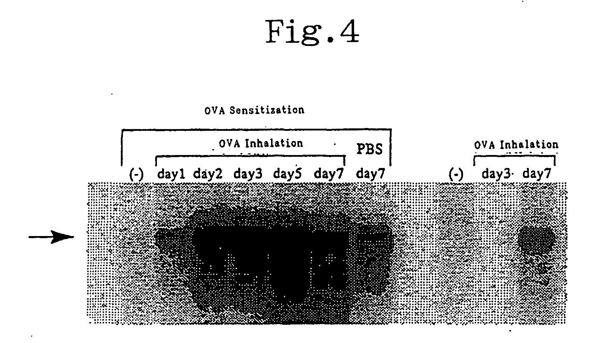

Using the airway hyperresponsive model mouse explained in EXAMPLE 1 above, the increased airway hyperresponsiveness and the count of infiltrated cells into alveolar lavage fluids were measured before OVA inhalation and on Days 2, 3, 4, 5, 6 and 7 after OVA inhalation as in EXAMPLE 1 (FIGS. 2 and 3). In addition, the lung before OVA inhalation and on Days 1, 2, 3, 5 and 7 after OVA inhalation was isolated, and subjected to Northern blotting analysis as in EXAMPLE 2 (FIG. 4). As a result, the increased airway hyperresponsiveness and infiltration of eosinophils into alveolar lavage fluids were induced on or after Day 4 after OVA inhalation, whereas expression of the Gob-5 gene was markedly induced from Day 2 after OVA inhalation. That is, expression of the Gob-5 gene occurred prior to the increased airway hyperresponsiveness and eosinophil infiltration, suggesting the possibility that the Gob-5 gene was not expressed as the outcome of airway inflammation and that induction of the Gob-5 gene expression would cause the increased airway hyperresponsiveness and eosinophil infiltration into alveolar lavage fluids.

### EXAMPLE 4

### Identification of the Gob-5 gene expression site in model mouse with increased airway hyperresponsiveness

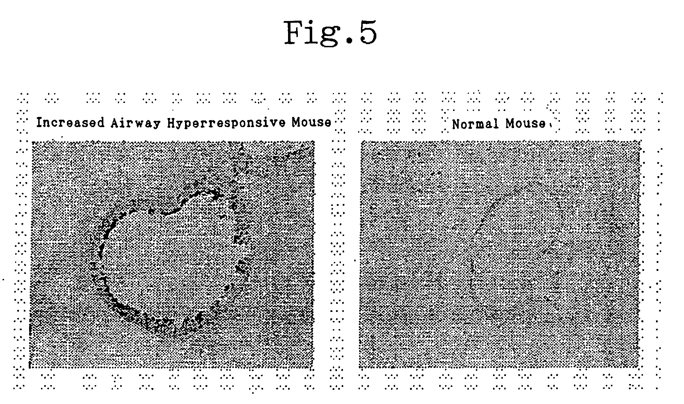

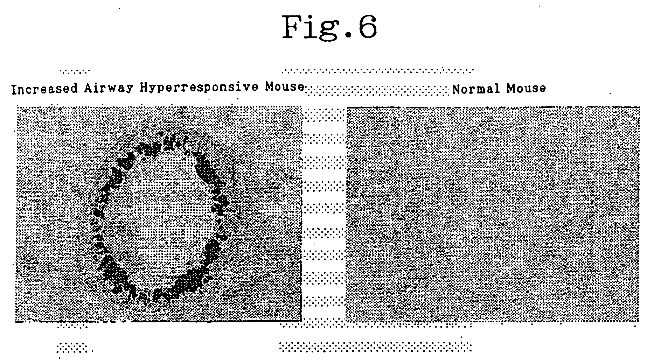

After perfusion and fixation in the lung of normal mouse and model mouse with increased airway hyperresponsiveness with 4% paraformaldehyde, the lung was isolated and fixed at 4°C overnight. Thereafter, a sucrose-HBSS solution was replaced by gradually increasing the concentration of sucrose to finally reach 18% sucrose-HBSS solution, and the lung was frozen in dry ice. After allowing to stand in a cryostat at -14°C for 3 hours, the frozen lung was cut into a thickness of 10-15 µl and put up on an APS-coated slide glass. For preparing a DIG-labeled probe, the Gob-5 DNA fragment of 0.5 kb was amplified by PCR using the Gob-5 full-length gene fragment obtained in EXAMPLE 1 as a template and using synthetic primers (PR2: SEQ ID NO: 6, PR3: SEQ IDNO: 7). Using Takara EX Taq (manufactured by Takara Shuzo Co., Ltd.), the reaction was carried out by initially reacting at 94°C for 1 minute and then repeating 30 cycles in Thermal Cycler Gene Amp PCR System 9700 (manufactured by Perkin-Elmer, Inc.), in which one cycle was set to include 94°C for 10 seconds, 60°C for 30 seconds and then 72°C for 90 seconds, and finally by reacting at 72°C for 10 minutes. The amplified DNA fragment was inserted into PCRII-TOPO vector (manufactured by Invitrogen, Inc.), and extended from both sides of the vector by SP6 RNA polymerase and T7 RNA polymerase using DIG Labeling Kit (manufactured by Boehringer Mannheim) in accordance with the manual attached to prepare DIG-labeled antisense and sense probe. In situ hybridization was conducted using ISHR Starter Kit (manufactured by Nippon Gene Co., Ltd.) in accordance with the manual attached thereto. As a result, it was found that the Gob-5 gene was highly expressed at the site around bronchi in the model animal with increased airway hyperresponsiveness. In the normal mouse, no expression of the Gob-5 gene was observed in any part (FIG. 5). It was reported that the Gob-5 gene was expressed on goblet cells in the small and large intestines [Biochem. Biophys. Res. Commun., 255, 347 (1999)]. In the bronchi of normal mouse, goblet cells are hardly present, but it is reported that goblet cells are overly expressed in the model mouse with increased airway hyperresponsiveness [Am. J. Respir. Cell Mol. Biol., 14, 425 (1996)]. Therefore, goblet cells were specifically stained by PAS staining to see if the expression of Gob-5 gene around the bronchi was attributable to goblet cells. PAS staining was performed by fixing each slice of the lung from normal mouse and from model mouse with increased airway hyperresponsiveness with 10% formalin solution for 10 minutes, oxidizing with 0.5% periodic acid aqueous solution for 5 minutes, treating with Schiff's base for 15 minutes and with an aqueous sulfurous acid solution for 3 minutes, dehydrating with ethanol, and penetrating with lemosol A. The results indicate that the stained sites of goblet cells around the bronchi well coincided with the Gob-5 expression sites, and goblet cells of the bronchi are considered to be Gob-5 expressing cells (FIG. 6).

### EXAMPLE 5

### Acquisition of CLCA1 gene as a human counterpart to the Gob-5 gene

Using the Geneble database, homology search of the Gob-5 gene by blast N was performed. The results reveal that the gene belongs to the calcium-dependent chloride channel family, showing extremely high homology to human CLCA1 gene [Genomics, 54, 200 (1998)], which was 72% on a base level and 74% on an amino acid level. Since the CLCA1 gene shows properties similar to those of the Gob-5 gene in that the gene is tissue-dependently expressed in the small/large intestines in healthy subject and expressed strongly in goblet cells present in the intestine, human counterpart of Gob-5 was assumed to be CLCA1.PCR was thus conducted using human small intestine cDNA library (manufactured by Clontech Laboratories, Inc.) as a template and two primer DNAs of CLCA1 gene 5'-non-translational region (PR18: SEQ ID NO: 24) and 3'-non-translational region (PR19: SEQ ID NO: 25) to obtain CLCA1 full-length gene (SEQ ID NO: 26). Using Takara EX Taq (manufactured by Takara Shuzo Co., Ltd.), the reaction was carried out by initially reacting at 98°C for 1 minute and then repeating 30 cycles in Thermal Cycler Gene Amp PCR System 9700 (manufactured by Perkin-Elmer, Inc.), in which one cycle was set to include 98°C for 10 seconds, 60°C for 1 minutes and then 72°C for 3 minutes, and finally by reacting at 72°C for 10 minutes. The amplified DNA fragment was inserted into pCRII-TOPO vector (manufactured by Invitrogen, Inc.). Cycle sequencing was further conducted using synthetic primers (PR20 to PR 31: SEQ ID NO: 27 to SEQ ID NO: 38) to identify the base sequence of the reaction product by a fluorescent DNA sequencer (ABI PRISMTM377,manufactured by Perkin-Elmer, Inc.).

### EXAMPLE 6

### Construction of vector to express human CLCA1 gene in animal cells

pCRII-TOPO Vector inserted with the human CLCA1 gene shown in EXAMPLE 5 was digested with KpnI-NotI. The resulting DNA fragment of 2.9 kbp containing the CLCA1 gene was inserted into pcDNA 3.1 plasmid (manufactured by Invitrogen, Inc.) similarly digested with KpnI-NotI to construct plasmid pcDNA-hCLCA1 bearing the human CLCA1 gene downstream cytomegalovirus enhancer/promoter and having a neomycin resistant gene as a selection marker.

### EXAMPLE 7

### Suppression of the increased airway hyperresponsiveness by administration of the antisense oligonucleotide to the Gob-5 gene

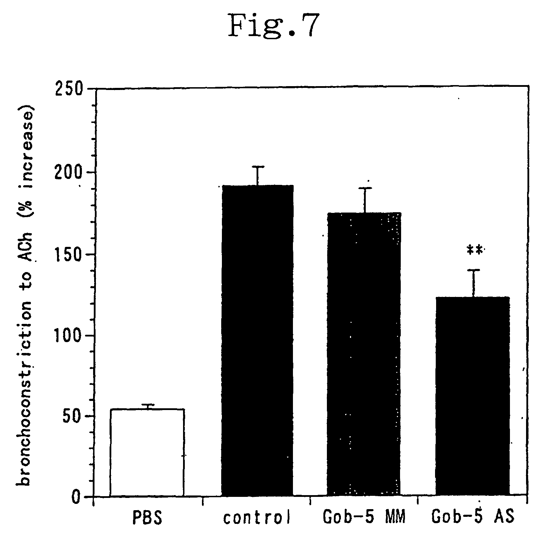

To clarify that expression of the Gob-5 gene would be associated with increased airway hyperresponsiveness, the effect on airway reactivity was examined by administering phosphorothioate type antisense oligonucleotide (Gob-SAS: SEQ ID NO: 39) near the translation initiation site of the Gob-5 gene and mismatched mutation phosphorothioate type antisense oligonucleotide (Gob-5MM: SEQ ID NO: 40) for control. In the model mouse with increased airway hyperresponsiveness shown in EXAMPLE 1, Gob-5AS and Gob-5MM suspended, respectively, in physiological saline (1 mg/kg) was intrabronchially given for 7 consecutive days an hour before and 4 hours after OVA inhalation. The airway reactivity was determined by the method shown in EXAMPLE 1. The results indicate that the increased airway hyperresponsiveness was significantly suppressed by administration of Gob-5AS but no significant suppression was noted with Gob-5MM for control (FIG. 7), clearly showing that the Gob-5 gene was associated with the increased airway hyperresponsiveness.

### EXAMPLE 8

### Construction of virus vector for expressing antisense strand RNA to Gob-5 gene

The Gob-5 gene-inserted pT7 Blue-T Vector shown in EXAMPLE 1 was digested with HincII-SmaI. The resulting DNA fragment of 2.9 kbp containing the Gob-5 gene was inserted into SwaI-digested pAxCAwt cosmid vector (manufactured by Takara Shuzo Co., Ltd.) to construct cosmid vector pAxCA-Gob5-AS inversely inserted with the Gob-5 gene at the downstream of CAG promoter. This vector was transfected to 293 cells with restriction enzyme-treated DNA-TPC, in accordance with the attached manual, using Adenovirus Expression Vector Kit (manufactured by Takara Shuzo Co., Ltd.) to produce recombinant adenovirus AdV Gob-5-AS. Furthermore, the virus solution obtained by repeating infection to 293 cells 4 times was desalted and condensed using CentriconPlus-20 (manufactured by Millipore Corporation) to produce the virus solution having a high titer of 2.6 x 10¹¹ PFU/ml. For control, recombinant adenovirus AdV-wt was produced from cosmid vector pAxCAwt in a similar manner to acquire the high titer virus solution of 6.6 x 10¹⁰ PFU/ml.

### EXAMPLE 9

### Suppression of the increased airway hyperresponsiveness by administration of a virus vector for expressing the antisense strand RNA to the Gob-5 gene

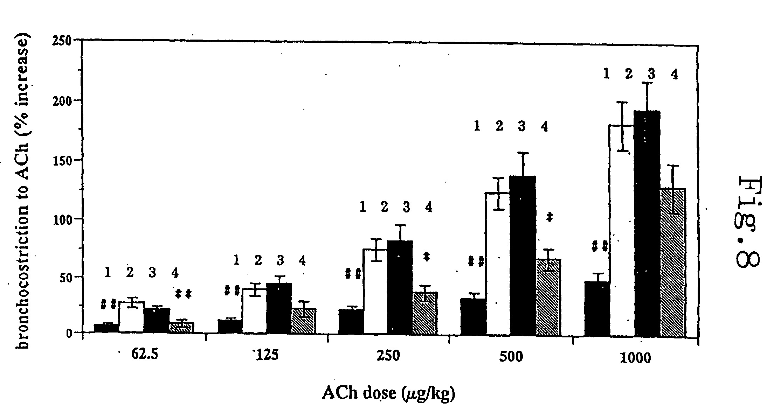

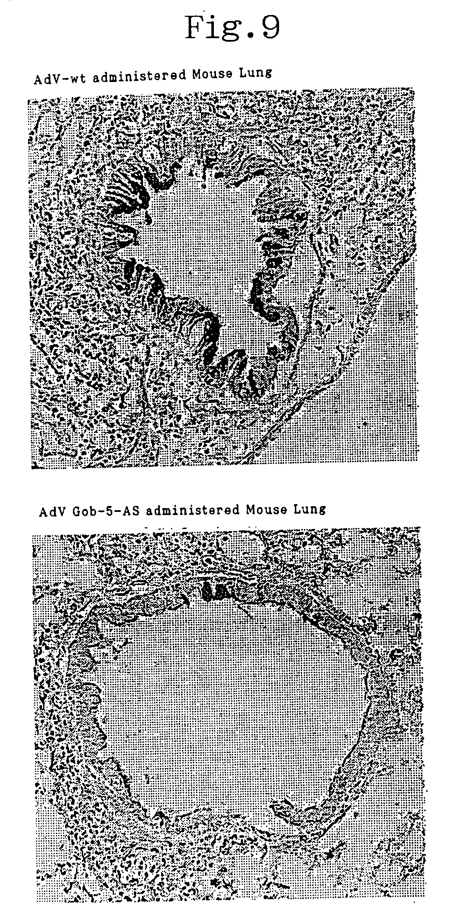

To confirm that expression of the Gob-5 gene would be associated with increased airway hyperresponsiveness shown in EXAMPLE 7, the effect on airway reactivity was examined by administering a virus vector for expressing the antisense strand RNA to the Gob-5 gene shown in EXAMPLE 8. In the model mouse with increased airway hyperresponsiveness shown in EXAMPLE 1, AdV Gob-5-AS and AdV-wt (5 x 10⁸ PFU/40 µl) diluted with distilled water was intrabronchially given one day before OVA inhalation. The airway reactivity was determined by the method shown in EXAMPLE 1. The results indicate that the increased airway hyperresponsiveness was significantly suppressed by administration of AdV Gob-5-AS but no significant suppression was noted with AdV-wt for control (FIG. 8). Thus, association of the Gob-5 gene with increased airway hyperresponsiveness was confirmed. Further after the airway reactivity assay, the lung was withdrawn from these mice and frozen slices were prepared in accordance with the procedure shown in EXAMPLE 4 for histological examination. In the airway epithelium of the AdV gob-5-AS-administered mouse, the count of PAS staining-positive goblet cells associated with mucous secretion was markedly reduced as compared to the control mouse administered with AdV-wt. The results indicate that Gob-5 takes part in differential growth/mucous secretion of goblet cells (FIG. 9).

### EXAMPLE 10

### Assay for Cl⁻ ion secretion using human CLCA1-expressing CHO cells

Human CLCA1-expressing plasmid pcDNA-hCLCA1 shown in EXAMPLE 6 was transfected to CHO cells using Gene transfer lyo (manufactured by Wako Pure Chemical Industries, Ltd.) in accordance with the attached manual. The cells were subjected to selective culture for 10 days in MEM-α medium (manufactured by GIBCO BRL) supplemented with 1 mg/ml of neomycin (G418) (manufactured by GIBCO BRL) to obtain the cells grown as human CLCA1 expressing CHO cells. The human CLCA1 expressing CHO cells and parent CHO cells were seeded on a 96-well plate (manufactured by Packard Inc.) coated with 2 µg/ml of fibronectin (manufactured by Itoham Foods, Inc.), respectively, in a concentration of 4 x 10⁴ cells/well, followed by culturing at 37°C for 24 hours under 5% CO₂. After completion, the culture solution was washed once with a buffer solution containing no Cl⁻ ions (2.4 mM K₂HPO₄, 0.6 mM KH₂PO₄, 10 mM D-glucose, 1 mM MgSO₄, 1 mM CaSO₄, 135 mM NaNO₃, 10 mM HEPES, pH 7.4), and 1 mM of Cl⁻ ion fluorescence indicator 6-methoxy-N-ethylquinolinium iodide (MEQ, manufactured by Moelcular Probes, Inc.) was added in an amount of 100 µl/well to the culture solution, followed by incubation at 37°C for further 2 hours. The culture solution was further washed twice with a Cl⁻ ion-containing buffer solution (2.4 mM K₂HPO₄, 0.6 mM KH₂PO₄, 10 mM D-glucose, 1 mM MgSO₄, 1 mM CaSO₄, 135 mM NaCl, 10 mM HEPES, pH 7.4), and then allowed to stand at room temperature for 30 minutes. Each well was replaced by the buffer solution containing no Cl⁻ ions and ionomycin was added to make the concentration of 10 µM. Immediately thereafter, the fluorescence intensity was measured at an excited wavelength of 355 nm and a measurement wavelength of 460 nm, usingamulti-label counter Wallac1420ARVOsx (manufactured by Amersham Pharmacia Biotech).

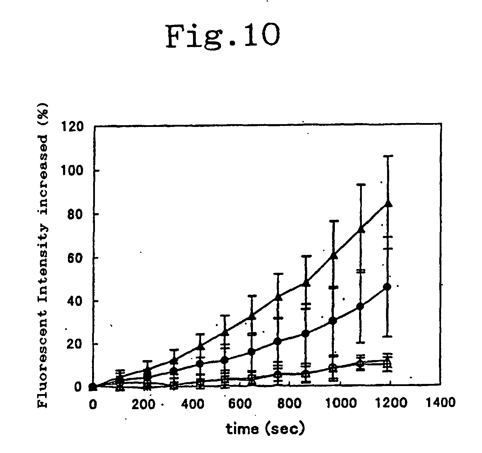

In either parent CHO cells or human CLCA1 expressing CHO cells, little increase in fluorescence intensity was noted in the state with no stimulation. When stimulated with 10 µM ionomycin (manufactured by Wako Pure Chemical Industries, Inc.), the fluorescence intensity increased in human CLCA1 expressing CHO cells by about twice, as compared to the parent CHO cells (FIG. 10). The results indicate that CLCA1 functions as Ca²⁺-dependent Cl⁻ ion channel.

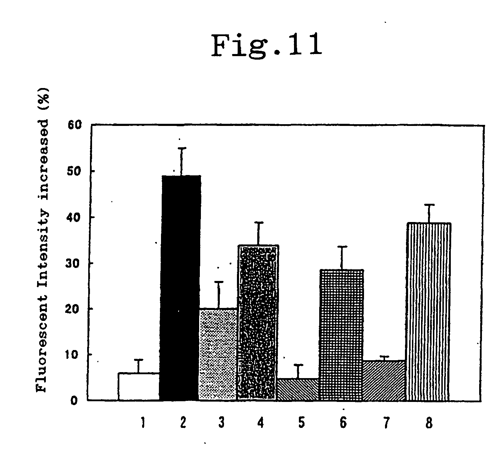

In the above experimental procedure, the culture solution was washed twice with the Cl⁻ - ion-containing buffer solution, and added an anionic transport inhibitor niflumic acid (NFA, manufactured by Cayman Chemical Co.), 4,4'-diisothiocyanastilbene-2,2'-disulfonic acid (DIDS, manufactured by Molecular Probes, Inc.) or dithiothreitol (DTT, manufactured by Wako Pure Chemical Industries, Inc.), and the mixture was allowed to stand at room temperature for 30 minutes. In this case, an increase of the fluorescence intensity by ionomycin stimulation of human CLCA1 expressing CHO cells was suppressed (FIG. 11).

The foregoing reveals that compounds or salts thereof that have the Cl⁻ ion channel inhibiting activity can be screened using the human CLCA1 expressing CHO cells. The compounds or salts thereof obtainable by this screening can be used for the prevention and/or treatment of, e.g., bronchial asthma, chronic obstructive pulmonary disease, etc.

### INDUSTRIAL APPLICABILITY

The protein having the same or substantially the same amino acid sequence as the amino acid sequence represented by SEQ ID NO: 1 is a diagnostic marker for bronchial asthma or chronic obstructive pulmonary disease, and therefore, the compound or its salts capable of inhibiting the activity of the protein, and the neutralizing antibodies that inhibit the activity of the protein can be used for the preventive/remedy for, e.g., bronchial asthma or chronic obstructive pulmonary disease, etc.

The antisense nucleotide of the present invention can suppress the expression of the protein having the same or substantially the same amino acid sequence as the amino acid sequence represented by SEQ ID NO: 1, and can thus be used for the preventive/remedy for, e.g., bronchial asthma or chronic obstructive pulmonary disease, etc.

## Claims

1. An antisense nucleotide having a base sequence complementary or substantially complementary to a base sequence of a DNA encoding a protein containing the same or substantially the same amino acid sequence as the amino acid sequence represented by SEQ ID NO: 1, or its partial peptide.

2. The antisense nucleotide according to Claim 1, wherein substantially the same amino acid sequence as the amino acid sequence represented by SEQ ID NO: 1 is the amino acid sequence represented by SEQ ID NO: 2.

3. The antisense nucleotide according to Claim 1, which has a base sequence complementary to the base sequence represented by SEQ ID NO: 3 or SEQ ID NO: 4, or a part thereof.

4. A pharmaceutical comprising the antisense nucleotide according to Claim 1.

5. The pharmaceutical according to Claim 4, which is for the prevention/treatment of bronchial asthma or chronic obstructive pulmonary disease.

6. A diagnostic comprising an antibody against a protein containing the same or substantially the same amino acid sequence as the amino acid sequence represented by SEQ ID NO: 1. its partial peptide, or a salt thereof.

7. A method of screening a compound or a salt thereof that inhibits the activity of a protein containing the same or substantially the same amino acid sequence as the amino acid sequence represented by SEQ IDNO: 1, its partial peptide. or a salt thereof, which comprises using a protein containing the same or substantially the same amino acid sequence as the amino acid sequence represented by SEQ ID NO: 1, its partial peptide, or a salt thereof.

8. The method of screening according to Claim 7, wherein the protein containing the same or substantially the same amino acid sequence as the amino acid sequence represented by SEQ ID NO: 1, its partial peptide, or a salt thereof is the one expressed on cell membrane of the transformant transformed with a DNA containing a DNA encoding the protein containing the same or substantially the same amino acid sequence as the amino acid sequence represented by SEQ ID NO: 1, its partial peptide, or a salt thereof.

9. A kit for screening a compound or a salt thereof that inhibits the activity of a protein containing the same or substantially the same amino acid sequence as the amino acid sequence represented by SEQ ID NO: 1, its partial peptide, or a salt thereof, comprising a protein containing the same or substantially the same amino acid sequence as the amino acid sequence represented by SEQ ID NO: 1, its partial peptide, or a salt thereof.

10. A compound or a salt thereof that inhibits the activity of a protein containing the same or substantially . the same amino acid sequence as the amino acid sequence represented by SEQ ID NO: 1, its partial peptide, or a salt thereof, which is obtainable using the screening method according to Claim 7 or the screening kit according to Claim 9.

11. A pharmaceutical comprising a compound or a salt thereof that inhibits the activity of a protein containing the same or substantially the same amino acid sequence as the amino acid sequence represented by SEQ ID NO: 1, its partial peptide, or a salt thereof, which is obtainable using the screening method according to Claim 7 or the screening kit according to Claim 9.

12. The pharmaceutical according to claim 11, which is for the prevention/treatment of bronchial asthma or chronic obstructive pulmonary disease.

13. A preventive/remedy for bronchial asthma or chronic obstructive pulmonary disease, comprising a compound having a calcium-dependent chloride channel inhibiting activity, or a salt thereof.

14. A method for the prevention/treatment of bronchial asthma or chronic obstructive pulmonary disease, which comprises administering the antisense nucleotide according to Claim 1 to a mammal.

15. Use of the antisense nucleotide according to Claim 1 formanufacturing a preventive/remedy for bronchial asthma or chronic obstructive pulmonary disease.

16. A method for the prevention/treatment of bronchial asthma or chronic obstructive pulmonary disease, which comprises administering to a mammal a compound or a salt thereof that inhibits the activity of a protein containing the same or substantially the same amino acid sequence as the amino acid sequence represented by SEQ ID NO: 1, its partial peptide, or a salt thereof, which is obtainable using the screening method according to Claim 7 or the screening kit according to Claim 9.

17. Use of a compound or a salt thereof that inhibits the activity of a protein containing the same or substantially the same amino acid sequence as the amino acid sequence represented by SEQ ID NO: 1, its partial peptide, or a salt thereof, which is obtainable using the screening method according to Claim 7 or the screening kit according to Claim 9, for manufacturing a preventive/remedy for bronchial asthma or chronic obstructive pulmonary disease.

18. A method for prevention/treatment of bronchial asthma or chronic obstructive pulmonary disease, which comprises administering a compound having a calcium-dependent chloride channel inhibiting activity, or a salt thereof to a mammal.

19. Use of a compound or its salt having a calcium-dependent chloride channel inhibiting activity, for manufacturing a preventive/remedy for bronchial asthma or chronic obstructive pulmonary disease.


SEQUENCE LISTING

<110> Takeda Chemical Industries, Ltd.
<120> Use of Disease-Related Gene
<130> 2672WOOP
<150> JP 11-333479
<151> 1999-11-24
<150> JP 2000-127589
<151> 2000-04-27
<160> 40
<210> 1
<211> 913
<212> PRT
<213> Mouse
<400> 1
Met Glu Ser Leu Lys Ser Pro Val Phe Leu Leu Ile Leu His Leu Leu
                5                   10                  15
Glu Gly Val Leu Ser Glu Ser Leu Ile Gln Leu Asn Asn Asn Gly Tyr
            20                  25                  30
Glu Gly Ile Val Ile Ala Ile Asp His Asp Val Pro Glu Asp Glu Ala
        35                  40                  45
Leu Ile Gln His Ile Lys Asp Met Val Thr Gln Ala Ser Pro Tyr Leu
    50                  55                  60
Phe Glu Ala Thr Gly Lys Arg Phe Tyr Phe Lys Asn Val Ala Ile Leu
65                  70                  75                  80
Ile Pro Glu Ser Trp Lys Ala Lys Pro Glu Tyr Thr Arg Pro Lys Leu
                85                  90                  95
Glu Thr Phe Lys Asn Ala Asp Val Leu Val Ser Thr Thr Ser Pro Leu
            100                 105                 110

Gly Asn Asp Glu Pro Tyr Thr Glu His Ile Gly Ala Cys Gly Glu Lys
        115                 120                 125
Gly Ile Arg Ile His Leu Thr Pro Asp Phe Leu Ala Gly Lys Lys Leu
    130                 135                 140
Thr Gln Tyr Gly Pro Gln Asp Arg Thr Phe Val His Glu Trp Ala His
145                 150                 155                 160
Phe Arg Trp Gly Val Phe Asn Glu Tyr Asn Asn Asp Glu Lys Phe Tyr
                165                 170                 175
Leu Ser Lys Gly Lys Pro Gln Ala Val Arg Cys Ser Ala Ala Ile Thr
            180                 185                 190
Gly Lys Asn Gln Val Arg Arg Cys Gln Gly Gly Ser Cys Ile Thr Asn
        195                 200                 205
Gly Lys Cys Val Ile Asp Arg Val Thr Gly Leu Tyr Lys Asp Asn Cys
    210                 215                 220
Val Phe Val Pro Asp Pro His Gln Asn Glu Lys Ala Ser Ile Met Phe
225                 230                 235                 240
Asn Gln Asn Ile Asn Ser Val Val Glu Phe Cys Thr Glu Lys Asn His
                245                 250                 255
Asn Gln Glu Ala Pro Asn Asp Gln Asn Gln Arg Cys Asn Leu Arg Ser
            260                 265                 270
Thr Trp Glu Val Ile Gln Glu Ser Glu Asp Phe Lys Gln Thr Thr Pro
        275                 280                 285
Met Thr Ala Gln Pro Pro Ala Pro Thr Phe Ser Leu Leu Gln Ile Gly
    290                 295                 300
Gln Arg Ile Val Cys Leu Val Leu Asp Lys Ser Gly Ser Met Leu Asn
305                 310                 315                 320
Asp Asp Arg Leu Asn Arg Met Asn Gln Ala Ser Arg Leu Phe Leu Leu
                325                 330                 335
Gln Thr Val Glu Gln Gly Ser Trp Val Gly Met Val Thr Phe Asp Ser

            340                 345                 350
Ala Ala Tyr Val Gln Ser Glu Leu Lys Gln Leu Asn Ser Gly Ala Asp
        355                 360                 365
Arg Asp Leu Leu Ile Lys His Leu Pro Thr Val Ser Ala Gly Gly Thr
    370                 375                 380
Ser Ile Cys Ser Gly Leu Arg Thr Ala Phe Thr Val Ile Lys Lys Lys
385                 390                 395                 400
Tyr Pro Thr Asp Gly Ser Glu Ile Val Leu Leu Thr Asp Gly Glu Asp
                405                 410                 415
Asn Thr Ile Ser Ser Cys Phe Asp Leu Val Lys Gln Ser Gly Ala Ile
            420                 425                 430
Ile His Thr Val Ala Leu Gly Pro Ala Ala Ala Lys Glu Leu Glu Gln
        435                 440                 445
Leu Ser Lys Met Thr Gly Gly Leu Gln Thr Tyr Ser Ser Asp Gln Val
    450                 455                 460
Gln Asn Asn Gly Leu Val Asp Ala Phe Ala Ala Leu Ser Ser Gly Asn
465                 470                 475                 480
Ala Ala Ile Ala Gln His Ser Ile Gln Leu Glu Ser Arg Gly Val Asn
                485                 490                 495
Leu Gln Asn Asn Gln Trp Met Asn Gly Ser Val Ile Val Asp Ser Ser
            500                 505                 510
Val Gly Lys Asp Thr Leu Phe Leu Ile Thr Trp Thr Thr His Pro Pro
        515                 520                 525
Thr Ile Phe Ile Trp Asp Pro Ser Gly Val Glu Gln Asn Gly Phe Ile
    530                 535                 540
Leu Asp Thr Thr Thr Lys Val Ala Tyr Leu Gln Val Pro Gly Thr Ala
545                 550                 555                 560
Lys Val Gly Phe Trp Lys Tyr Ser Ile Gln Ala Ser Ser Gln Thr Leu
                565                 570                 575

Thr Leu Thr Val Thr Ser Arg Ala Ala Ser Ala Thr Leu Pro Pro Ile
            580                 585                 590
Thr Val Thr Pro Val Val Asn Lys Asn Thr Gly Lys Phe Pro Ser Pro
        595                 600                 605
Val Thr Val Tyr Ala Ser Ile Arg Gln Gly Ala Ser Pro Ile Leu Arg
    610                 615                 620
Ala Ser Val Thr Ala Leu Ile Glu Ser Val Asn Gly Lys Thr Val Thr
625                 630                 635                 640
Leu Glu Leu Leu Asp Asn Gly Ala Gly Ala Asp Ala Thr Lys Asn Asp
                645                 650                 655
Gly Val Tyr Ser Arg Phe Phe Thr Ala Phe Asp Ala Asn Gly Arg Tyr
            660                 665                 670
Ser Val Lys Ile Trp Ala Leu Gly Gly Val Thr Ser Asp Arg Gln Arg
        675                 680                 685
Ala Ala Pro Pro Lys Asn Arg Ala Met Tyr Ile Asp Gly Trp Ile Glu
    690                 695                 700
Asp Gly Glu Val Arg Met Asn Pro Pro Arg Pro Glu Thr Ser Tyr Val
705                 710                 715                 720
Gln Asp Lys Gln Leu Cys Phe Ser Arg Thr Ser Ser Gly Gly Ser Phe
                725                 730                 735
Val Ala Thr Asn Val Pro Ala Ala Ala Pro Ile Pro Asp Leu Phe Pro
            740                 745                 750
Pro Cys Gln Ile Thr Asp Leu Lys Ala Ser Ile Gln Gly Gln Asn Leu
        755                 760                 765
Val Asn Leu Thr Trp Thr Ala Pro Gly Asp Asp Tyr Asp His Gly Arg
    770                 775                 780
Ala Ser Asn Tyr Ile Ile Arg Met Ser Thr Ser Ile Val Asp Leu Arg
785                 790                 795                 800
Asp His Phe Asn Thr Ser Leu Gln Val Asn Thr Thr Gly Leu Ile Pro

                805                 810                 815
Lys Glu Ala Ser Ser Glu Glu Ile Phe Glu Phe Glu Leu Gly Gly Asn
            820                 825                 830
Thr Phe Gly Asn Gly Thr Asp Ile Phe Ile Ala Ile Gln Ala Val Asp
        835                 840                 845
Lys Ser Asn Leu Lys Ser Glu Ile Ser Asn Ile Ala Arg Val Ser Val
    850                 855                 860
Phe Ile Pro Ala Gln Glu Pro Pro Ile Pro Glu Asp Ser Thr Pro Pro
865                 870                 875                 880
Cys Pro Asp Ile Ser Ile Asn Ser Thr Ile Pro Gly Ile His Val Leu
                885                 890                 895
Lys Ile Met Trp Lys Trp Leu Gly Glu Met Gln Val Thr Leu Gly Leu
            900                 905                 910
His
913

<210> 2
<211> 914
<212> PRT
<213> Human
<400> 2
Met Gly Pro Phe Lys Ser Ser Val Phe Ile Leu Ile Leu His Leu Leu
                5                   10                  15
Glu Gly Ala Leu Ser Asn Ser Leu Ile Gln Leu Asn Asn Asn Gly Tyr
            20                  25                  30
Glu Gly Ile Val Val Ala Ile Asp Pro Asn Val Pro Glu Asp Glu Thr
        35                  40                  45
Leu Ile Gln Gln Ile Lys Asp Met Val Thr Gln Ala Ser Leu Tyr Leu
    50                  55                  60

Phe Glu Ala Thr Gly Lys Arg Phe Tyr Phe Lys Asn Val Ala Ile Leu
65                  70                  75                  80
Ile Pro Glu Thr Trp Lys Thr Lys Ala Asp Tyr Val Arg Pro Lys Leu
                85                  90                  95
Glu Thr Tyr Lys Asn Ala Asp Val Leu Val Ala Glu Ser Thr Pro Pro
            100                 105                 110
Gly Asn Asp Glu Pro Tyr Thr Glu Gln Met Gly Asn Cys Gly Glu Lys
        115                 120                 125
Gly Glu Arg Ile His Leu Thr Pro Asp Phe Ile Ala Gly Lys Lys Leu
    130                 135                 140
Ala Glu Tyr Gly Pro Gln Gly Lys Ala Phe Val His Glu Trp Ala His
145                 150                 155                 160
Leu Arg Trp Gly Val Phe Asp Glu Tyr Asn Asn Asp Glu Lys Phe Tyr
                165                 170                 175
Leu Ser Asn Gly Arg Ile Gln Ala Val Arg Cys Ser Ala Gly Ile Thr
            180                 185                 190
Gly Thr Asn Val Val Lys Lys Cys Gln Gly Gly Ser Cys Tyr Thr Lys
        195                 200                 205
Arg Cys Thr Phe Asn Lys Val Thr Gly Leu Tyr Glu Lys Gly Cys Glu
    210                 215                 220
Phe Val Leu Gln Ser Arg Gln Thr Glu Lys Ala Ser Ile Met Phe Ala
225                 230                 235                 240
Gln His Val Asp Ser Ile Val Glu Phe Cys Thr Glu Gln Asn His Asn
                245                 250                 255
Lys Glu Ala Pro Asn Lys Gln Asn Gln Lys Cys Asn Leu Arg Ser Thr
            260                 265                 270
Trp Glu Val Ile Arg Asp Ser Glu Asp Phe Lys Lys Thr Thr Pro Met
        275                 280                 285
Thr Thr Gln Pro Pro Asn Pro Thr Phe Ser Leu Leu Gln Ile Gly Gln

    290                 295                 300
Arg Ile Val Cys Leu Val Leu Asp Lys Ser Gly Ser Met Ala Thr Gly
305                 310                 315                 320
Asn Arg Leu Asn Arg Leu Asn Gln Ala Gly Gln Leu Phe Leu Leu Gln
                325                 330                 335
Thr Val Glu Leu Gly Ser Trp Val Gly Met Val Thr Phe Asp Ser Ala
            340                 345                 350
Ala His Val Gln Ser Glu Leu Ile Gln Ile Asn Ser Gly Ser Asp Arg
        355                 360                 365
Asp Thr Leu Ala Lys Arg Leu Pro Ala Ala Ala Ser Gly Gly Thr Ser
    370                 375                 380
Ile Cys Ser Gly Leu Arg Ser Ala Phe Thr Val Ile Arg Lys Lys Tyr
385                 390                 395                 400
Pro Thr Asp Gly Ser Glu Ile Val Leu Leu Thr Asp Gly Glu Asp Asn
                405                 410                 415
Thr Ile Ser Gly Cys Phe Asn Glu Val Lys Gln Ser Gly Ala Ile Ile
            420                 425                 430
His Thr Val Ala Leu Gly Pro Ser Ala Ala Gln Glu Leu Glu Glu Leu
        435                 440                 445
Ser Lys Met Thr Gly Gly Leu Gln Thr Tyr Ala Ser Asp Gln Val Gln
    450                 455                 460
Asn Asn Gly Leu Ile Asp Ala Phe Gly Ala Leu Ser Ser Gly Asn Gly
465                 470                 475                 480
Ala Val Ser Gln Arg Ser Ile Gln Leu Glu Ser Lys Gly Leu Thr Leu
                485                 490                 495
Gln Asn Ser Gln Trp Met Asn Gly Thr Val Ile Val Asp Ser Thr Val
            500                 505                 510
Gly Lys Asp Thr Leu Phe Leu Ile Thr Trp Thr Thr Gln Pro Pro Gln
        515                 520                 525

Ile Leu Leu Trp Asp Pro Ser Gly Gln Lys Gln Gly Gly Phe Val Val
    530                 535                 540
Asp Lys Asn Thr Lys Met Ala Tyr Leu Gln Ile Pro Gly Ile Ala Lys
545                 550                 555                 560
Val Gly Thr Trp Lys Tyr Ser Leu Gln Ala Ser Ser Gln Thr Leu Thr
                565                 570                 575
Leu Thr Val Thr Ser Arg Ala Ser Asn Ala Thr Leu Pro Pro Ile Thr
            580                 585                 590
Val Thr Ser Lys Thr Asn Lys Asp Thr Ser Lys Phe Pro Ser Pro Leu
        595                 600                 605
Val Val Tyr Ala Asn Ile Arg Gln Gly Ala Ser Pro Ile Leu Arg Ala
    610                 615                 620
Ser Val Thr Ala Leu Ile Glu Ser Val Asn Gly Lys Thr Val Thr Leu
625                 630                 635                 640
Glu Leu Leu Asp Asn Gly Ala Gly Ala Asp Ala Thr Lys Asp Asp Gly
                645                 650                 655
Val Tyr Ser Arg Tyr Phe Thr Thr Tyr Asp Thr Asn Gly Arg Tyr Ser
            660                 665                 670
Val Lys Val Arg Ala Leu Gly Gly Val Asn Ala Ala Arg Arg Arg Val
        675                 680                 685
Ile Pro Gln Gln Ser Gly Ala Leu Tyr Ile Pro Gly Trp Ile Glu Asn
    690                 695                 700
Asp Glu Ile Gln Trp Asn Pro Pro Arg Pro Glu Ile Asn Lys Asp Asp
705                 710                 715                 720
Val Gln His Lys Gln Val Cys Phe Ser Arg Thr Ser Ser Gly Gly Ser
                725                 730                 735
Phe Val Ala Ser Asp Val Pro Asn Ala Pro Ile Pro Asp Leu Phe Pro
            740                 745                 750
Pro Gly Gln Ile Thr Asp Leu Lys Ala Glu Ile His Gly Gly Ser Leu

        755                 760                 765
Ile Asn Leu Thr Trp Thr Ala Pro Gly Asp Asp Tyr Asp His Gly Thr
    770                 775                 780
Ala His Lys Tyr Ile Ile Arg Ile Ser Thr Ser Ile Leu Asp Leu Arg
785                 790                 795                 800
Asp Lys Phe Asn Glu Ser Leu Gln Val Asn Thr Thr Ala Leu Ile Pro
                805                 810                 815
Lys Glu Ala Asn Ser Glu Glu Val Phe Leu Phe Lys Pro Glu Asn Ile
            820                 825                 830
Thr Phe Glu Asn Gly Thr Asp Leu Phe Ile Ala Ile Gln Ala Val Asp
        835                 840                 845
Lys Val Asp Leu Lys Ser Glu Ile Ser Asn Ile Ala Arg Val Ser Leu
    850                 855                 860
Phe Ile Pro Pro Gln Thr Pro Pro Glu Thr Pro Ser Pro Asp Glu Thr
865                 870                 875                 880
Ser Ala Pro Cys Pro Asn Ile His Ile Asn Ser Thr Ile Pro Gly Ile
                885                 890                 895
His Ile Leu Lys Ile Met Trp Lys Trp Ile Gly Glu Leu Gln Leu Ser
            900                 905                 910
Ile Ala
914

<210> 3
<211> 2739
<212> DNA
<213> Mouse
<400> 3
ATGGAATCTT TGAAGAGTCC TGTCTTCCTC TTGATCCTCC ACCTTCTGGA AGGAGTTCTG 60
AGTGAGTCCC TCATCCAACT GAACAACAAC GGCTATGAGG GCATCGTCAT CGCCATAGAC 120

CACGACGTGC CGGAAGATGA AGCCCTCATT CAACACATAA AGGACATGGT GACTCAGGCC 180
TCTCCATACC TGTTTGAAGC TACAGGAAAA AGATTTTACT TCAAAAATGT TGCCATTTTG 240
ATTCCCGAGA GCTGGAAGGC AAAGCCTGAA TATACGAGGC CAAAACTTGA AACCTTCAAA 300
AACGCTGATG TCCTTGTATC AACAACCAGC CCTCTAGGCA ATGATGAGCC CTACACCGAA 360
CATATAGGAG CATGTGGAGA AAAGGGGATC AGGATTCACC TGACTCCTGA CTTCTTAGCA 420
GGAAAGAAGC TGACTCAGTA TGGGCCACAA GACAGGACCT TTGTCCATGA GTGGGCTCAC 480
TTCCGATGGG GAGTGTTTAA TGAATACAAC AACGACGAGA AGTTCTACTT ATCCAAAGGA 540
AAACCCCAAG CAGTGAGGTG TTCAGCAGCC ATTACCGGTA AAAATCAAGT TCGTCGGTGC 600
CAGGGAGGCA GTTGTATCAC TAACGGAAAG TGTGTAATCG ACAGAGTAAC GGGACTGTAT 660
AAAGACAATT GTGTATTTGT ACCAGATCCA CACCAAAACG AGAAGGCTTC CATCATGTTT 720
AACCAAAATA TCAATTCTGT GGTTGAATTC TGTACAGAAA AAAATCACAA TCAAGAAGCC 780
CCAAATGACC AAAACCAACG ATGCAATCTC CGAAGCACGT GGGAAGTCAT CCAGGAATCT 840
GAGGACTTCA AGCAAACCAC TCCCATGACA GCCCAGCCAC CTGCACCCAC CTTCTCACTG 900
CTGCAAATTG GACAAAGAAT TGTGTGCTTA GTTCTTGATA AGTCCGGGAG CATGCTGAAC 960
GATGATCGTC TTAACCGAAT GAATCAGGCA AGCCGGCTTT TCCTGCTGCA GACTGTGGAG 1020
CAGGGATCCT GGGTCGGGAT GGTGACCTTT GACAGTGCTG CCTATGTACA AAGCGAACTC 1080
AAACAGTTAA ACAGTGGTGC TGACAGAGAT CTGCTGATCA AGCACTTACC CACAGTATCT 1140
GCAGGAGGGA CATCTATATG CTCTGGCCTT CGGACAGCAT TTACAGTGAT AAAGAAGAAG 1200
TATCCAACTG ATGGATCTGA AATTGTGCTG CTGACCGATG GGGAGGACAA CACCATTAGC 1260
AGCTGCTTTG ACCTGGTGAA GCAGAGCGGG GCCATCATCC ATACAGTGGC CCTGGGACCG 1320
GCTGCCGCTA AAGAGCTTGA GCAGCTGTCC AAAATGACAG GAGGCCTGCA GACATACTCT 1380
TCGGATCAGG TTCAGAACAA TGGTCTTGTT GATGCTTTCG CAGCACTCTC CTCAGGAAAT 1440
GCGGCGATCG CTCAGCACTC CATCCAGCTG GAGAGCAGGG GAGTTAATCT CCAGAATAAC 1500
CAATGGATGA ATGGCTCAGT GATCGTGGAC AGCTCGGTGG GCAAGGACAC CTTGTTTCTT 1560
ATCACCTGGA CAACGCATCC TCCTACAATA TTTATCTGGG ATCCCAGCGG AGTGGAACAA 1620
AATGGTTTTA TACTAGACAC AACCACTAAG GTGGCCTACC TCCAAGTCCC AGGCACGGCT 1680
AAGGTTGGCT TTTGGAAATA CAGCATTCAA GCGAGCTCAC AGACTCTCAC CTTGACTGTC 1740
ACCTCCCGTG CAGCAAGTGC TACACTGCCT CCTATTACAG TGACCCCGGT AGTGAATAAG 1800
AACACAGGGA AATTCCCCAG CCCTGTAACA GTGTATGCAA GCATTCGCCA AGGAGCCTCG 1860

CCTATTCTCA GGGCCAGCGT CACAGCCTTG ATTGAATCTG TGAATGGAAA AACAGTAACC 1920
CTGGAATTAC TGGATAACGG AGCAGGTGCC GATGCCACCA AGAATGATGG TGTCTACTCA 1980
AGGTTTTTTA CAGCTTTTGA TGCAAATGGT AGATACAGCG TTAAAATATG GGCTCTGGGA 2040
GGAGTCACTT CAGACAGACA GAGAGCAGCA CCTCCGAAGA ACAGAGCCAT GTACATAGAT 2100
GGCTGGATTG AGGATGGTGA AGTAAGAATG AACCCACCAC GTCCTGAAAC TAGTTATGTT 2160
CAAGACAAGC AGCTGTGCTT CAGCAGGACA TCTTCAGGGG GATCGTTTGT GGCCACCAAT 2220
GTCCCCGCAG CAGCTCCCAT TCCTGACCTC TTTCCACCCT GTCAAATCAC TGACCTGAAG 2280
GCCAGCATCC AAGGGCAGAA CCTGGTGAAT CTGACGTGGA CGGCTCCTGG GGATGACTAC 2340
GACCACGGGA GAGCTTCCAA CTACATCATC CGAATGAGCA CCAGTATCGT TGATCTCAGG 2400
GACCACTTCA ACACCTCACT CCAAGTGAAC ACTACCGGTC TTATCCCCAA AGAGGCCAGC 2460
TCTGAGGAAA TCTTTGAGTT TGAACTGGGA GGCAACACTT TTGGAAATGG CACAGATATC 2520
TTCATTGCTA TCCAGGCTGT GGATAAGTCC AATCTGAAAT CAGAAATCTC CAACATTGCA 2580
CGGGTGTCTG TGTTCATCCC CGCTCAGGAG CCGCCCATTC CCGAAGACTC AACTCCCCCT 2640
TGTCCTGACA TCAGCATCAA CAGCACCATT CCTGGCATCC ACGTGCTGAA GATAATGTGG 2700
AAGTGGCTAG GGGAAATGCA GGTGACACTA GGTTTGCAC 2739

<210> 4
<211> 2742
<212> DNA
<213> Human
<400> 4
ATGGGGCCAT TTAAGAGTTC TGTGTTCATC TTGATTCTTC ACCTTCTAGA AGGGGCCCTG 60
AGTAATTCAC TCATTCAGCT GAACAACAAT GGCTATGAAG GCATTGTCGT TGCAATCGAC 120
CCCAATGTGC CAGAAGATGA AACACTCATT CAACAAATAA AGGACATGGT GACCCAGGCA 180
TCTCTGTATC TGTTTGAAGC TACAGGAAAG CGATTTTATT TCAAAAATGT TGCCATTTTG 240
ATTCCTGAAA CATGGAAGAC AAAGGCTGAC TATGTGAGAC CAAAACTTGA GACCTACAAA 300
AATGCTGATG TTCTGGTTGC TGAGTCTACT CCTCCAGGTA ATGATGAACC CTACACTGAG 360
CAGATGGGCA ACTGTGGAGA GAAGGGTGAA AGGATCCACC TCACTCCTGA TTTCATTGCA 420
GGAAAAAAGT TAGCTGAATA TGGACCACAA GGTAAGGCAT TTGTCCATGA GTGGGCTCAT 480

CTACGATGGG GAGTATTTGA CGAGTACAAT AATGATGAGA AATTCTACTT ATCCAATGGA 540
AGAATACAAG CAGTAAGATG TTCAGCAGGT ATTACTGGTA CAAATGTAGT AAAGAAGTGT 600
CAGGGAGGCA GCTGTTACAC CAAAAGATGC ACATTCAATA AAGTTACAGG ACTCTATGAA 660
AAAGGATGTG AGTTTGTTCT CCAATCCCGC CAGACGGAGA AGGCTTCTAT AATGTTTGCA 720
CAACATGTTG ATTCTATAGT TGAATTCTGT ACAGAACAAA ACCACAACAA AGAAGCTCCA 780
AACAAGCAAA ATCAAAAATG CAATCTCCGA AGCACATGGG AAGTGATCCG TGATTCTGAG 840
GACTTTAAGA AAACCACTCC TATGACAACA CAGCCACCAA ATCCCACCTT CTCATTGCTG 900
CAGATTGGAC AAAGAATTGT GTGTTTAGTC CTTGACAAAT CTGGAAGCAT GGCGACTGGT 960
AACCGCCTCA ATCGACTGAA TCAAGCAGGC CAGCTTTTCC TGCTGCAGAC AGTTGAGCTG 1020
GGGTCCTGGG TTGGGATGGT GACATTTGAC AGTGCTGCCC ATGTACAAAG TGAACTCATA 1080
CAGATAAACA GTGGCAGTGA CAGGGACACA CTCGCCAAAA GATTACCTGC AGCAGCTTCA 1140
GGAGGGACGT CCATCTGCAG CGGGCTTCGA TCGGCATTTA CTGTGATTAG GAAGAAATAT 1200
CCAACTGATG GATCTGAAAT TGTGCTGCTG ACGGATGGGG AAGACAACAC TATAAGTGGG 1260
TGCTTTAACG AGGTCAAACA AAGTGGTGCC ATCATCCACA CAGTCGCTTT GGGGCCCTCT 1320
GCAGCTCAAG AACTAGAGGA GCTGTCCAAA ATGACAGGAG GTTTACAGAC ATATGCTTCA 1380
GATCAAGTTC AGAACAATGG CCTCATTGAT GCTTTTGGGG CCCTTTCATC AGGAAATGGA 1440
GCTGTCTCTC AGCGCTCCAT CCAGCTTGAG AGTAAGGGAT TAACCCTCCA GAACAGCCAG 1500
TGGATGAATG GCACAGTGAT CGTGGACAGC ACCGTGGGAA AGGACACTTT GTTTCTTATC 1560
ACCTGGACAA CGCAGCCTCC CCAAATCCTT CTCTGGGATC CCAGTGGACA GAAGCAAGGT 1620
GGCTTTGTAG TGGACAAAAA CACCAAAATG GCCTACCTCC AAATCCCAGG CATTGCTAAG 1680
GTTGGCACTT GGAAATACAG TCTGCAAGCA AGCTCACAAA CCTTGACCCT GACTGTCACG 1740
TCCCGTGCGT CCAATGCTAC CCTGCCTCCA ATTACAGTGA CTTCCAAAAC GAACAAGGAC 1800
ACCAGCAAAT TCCCCAGCCC TCTGGTAGTT TATGCAAATA TTCGCCAAGG AGCCTCCCCA 1860
ATTCTCAGGG CCAGTGTCAC AGCCCTGATT GAATCAGTGA ATGGAAAAAC AGTTACCTTG 1920
GAACTACTGG ATAATGGAGC AGGTGCTGAT GCTACTAAGG ATGACGGTGT CTACTCAAGG 1980
TATTTCACAA CTTATGACAC GAATGGTAGA TACAGTGTAA AAGTGCGGGC TCTGGGAGGA 2040
GTTAACGCAG CCAGACGGAG AGTGATACCC CAGCAGAGTG GAGCACTGTA CATACCTGGC 2100
TGGATTGAGA ATGATGAAAT ACAATGGAAT CCACCAAGAC CTGAAATTAA TAAGGATGAT 2160
GTTCAACACA AGCAAGTGTG TTTCAGCAGA ACATCCTCGG GAGGCTCATT TGTGGCTTCT 2220

GATGTCCCAA ATGCTCCCAT ACCTGATCTC TTCCCACCTG GCCAAATCAC CGACCTGAAG 2280
GCGGAAATTC ACGGGGGCAG TCTCATTAAT CTGACTTGGA CAGCTCCTGG GGATGATTAT 2340
GACCATGGAA CAGCTCACAA GTATATCATT CGAATAAGTA CAAGTATTCT TGATCTCAGA 2400
GACAAGTTCA ATGAATCTCT TCAAGTGAAT ACTACTGCTC TCATCCCAAA GGAAGCCAAC 2460
TCTGAGGAAG TCTTTTTGTT TAAACCAGAA AACATTACTT TTGAAAATGG CACAGATCTT 2520
TTCATTGCTA TTCAGGCTGT TGATAAGGTC GATCTGAAAT CAGAAATATC CAACATTGCA 2580
CGAGTATCTT TGTTTATTCC TCCACAGACT CCGCCAGAGA CACCTAGTCC TGATGAAACG 2640
TCTGCTCCTT GTCCTAATAT TCATATCAAC AGCACCATTC CTGGCATTCA CATTTTAAAA 2700
ATTATGTGGA AGTGGATAGG AGAACTGCAG CTGTCAATAG CC 2742

<210> 5
<211> 22
<212> DNA
<213> Artificial Sequence
<220>
<223> Primer
<400> 5
GGGAAAGCTG CAGGATGGAA TC 22

<210> 6
<211> 26
<212> DNA
<213> Artificial Sequence
<220>
<223> Primer
<400> 6
TATACCGCCC CCTACAGGAA GTCTAA 26

<210> 7

<211> 21
<212> DNA
<213> Artificial Sequence
<220>
<223> Primer
<400> 7
CTACGACCAC GGGAGAGCTT C     21

<210> 8
<211> 21
<212> DNA
<213> Artificial Sequence
<220>
<223> Primer
<400> 8
GAAGCTCTCC CGTGGTCGTA G     21

<210> 9
<211> 24
<212> DNA
<213> Artificial Sequence
<220>
<223> Primer
<400> 9
CAATGATGAG CCCTACACCG AACA     24

<210> 10
<211> 24
<212> DNA

<213> Artificial Sequence

<220>

<223> Primer

<400> 10

TGTTCGGTGT AGGGCTCATC ATTG     24

<210> 11

<211> 21

<212> DNA

<213> Artificial Sequence

<220>

<223> Primer

<400> 11

ACCACTCCCA TGACAGCCCA G     21

<210> 12

<211> 21

<212> DNA

<213> Artificial Sequence

<220>

<223> Primer

<400> 12

CTGGGCTGTC ATGGGAGTGG T     21

<210> 13

<211> 22

<212> DNA

<213> Artificial Sequence

<220>

<223> Primer
<400> 13
TGGCCTTCGG ACAGCATTTA CA 22

<210> 14
<211> 22
<212> DNA
<213> Artificial Sequence
<220>
<223> Primer
<400> 14
TGTAAATGCT GTCCGAAGGC CA 22

<210> 15
<211> 24
<212> DNA
<213> Artificial Sequence
<220>
<223> Primer
<400> 15
ATGCGTTGTC CAGGTGATAA GAAA 24

<210> 16
<211> 27
<212> DNA
<213> Artificial Sequence
<220>
<223> Primer
<400> 16

GGTGGCCTAC CTCCAAGTCC CAGGCAC 27

<210> 17
<211> 28
<212> DNA
<213> Artificial Sequence
<220>
<223> Primer
<400> 17
CCCGTGCAGC AAGTGCTACA CTGCCTCC 28

<210> 18
<211> 28
<212> DNA
<213> Artificial Sequence
<220>
<223> Primer
<400> 18
GGAGGCAGTG TAGCACTTGC TGCACGGG 28

<210> 19
<211> 28
<212> DNA
<213> Artificial Sequence
<220>
<223> Primer
<400> 19
CGCCAAGGAG CCTCGCCTAT TCTCAGGG 28

<210> 20
<211> 27
<212> DNA
<213> Artificial Sequence
<220>
<223> Primer
<400> 20
AGGCTGTGAC GCTGGCCCTG AGAATAG     27

<210> 21
<211> 25
<212> DNA
<213> Artificial Sequence
<220>
<223> Primer
<400> 21
CTTGGTGGCA TCGGCACCTG CTCCG     25

<210> 22
<211> 2843
<212> DNA
<213> Mouse
<400> 22
GGGAAAGCTG CAGGATGGAA TCTTTGAAGA GTCCTGTCTT CCTCTTGATC CTCCACCTTC     60
TGGAAGGAGT TCTGAGTGAG TCCCTCATCC AACTGAACAA CAACGGCTAT GAGGGCATCG     120
TCATCGCCAT AGACCACGAC GTGCCGGAAG ATGAAGCCCT CATTCAACAC ATAAAGGACA     180
TGGTGACTCA GGCCTCTCCA TACCTGTTTG AAGCTACAGG AAAAAGATTT TACTTCAAAA     240
ATGTTGCCAT TTTGATTCCC GAGAGCTGGA AGGCAAAGCC TGAATATACG AGGCCAAAAC     300
TTGAAACCTT CAAAAACGCT GATGTCCTTG TATCAACAAC CAGCCCTCTA GGCAATGATG     360

AGCCCTACAC CGAACATATA GGAGCATGTG GAGAAAAGGG GATCAGGATT CACCTGACTC 420
CTGACTTCTT AGCAGGAAAG AAGCTGACTC AGTATGGGCC ACAAGACAGG ACCTTTGTCC 480
ATGAGTGGGC TCACTTCCGA TGGGGAGTGT TTAATGAATA CAACAACGAC GAGAAGTTCT 540
ACTTATCCAA AGGAAAACCC CAAGCAGTGA GGTGTTCAGC AGCCATTACC GGTAAAAATC 600
AAGTTCGTCG GTGCCAGGGA GGCAGTTGTA TCACTAACGG AAAGTGTGTA ATCGACAGAG 660
TAACGGGACT GTATAAAGAC AATTGTGTAT TTGTACCAGA TCCACACCAA AACGAGAAGG 720
CTTCCATCAT GTTTAACCAA AATATCAATT CTGTGGTTGA ATTCTGTACA GAAAAAAATC 780
ACAATCAAGA AGCCCCAAAT GACCAAAACC AACGATGCAA TCTCCGAAGC ACGTGGGAAG 840
TCATCCAGGA ATCTGAGGAC TTCAAGCAAA CCACTCCCAT GACAGCCCAG CCACCTGCAC 900
CCACCTTCTC ACTGCTGCAA ATTGGACAAA GAATTGTGTG CTTAGTTCTT GATAAGTCCG 960
GGAGCATGCT GAACGATGAT CGTCTTAACC GAATGAATCA GGCAAGCCGG CTTTTCCTGC 1020
TGCAGACTGT GGAGCAGGGA TCCTGGGTCG GGATGGTGAC CTTTGACAGT GCTGCCTATG 1080
TACAAAGCGA ACTCAAACAG TTAAACAGTG GTGCTGACAG AGATCTGCTG ATCAAGCACT 1140
TACCCACAGT ATCTGCAGGA GGGACATCTA TATGCTCTGG CCTTCGGACA GCATTTACAG 1200
TGATAAAGAA GAAGTATCCA ACTGATGGAT CTGAAATTGT GCTGCTGACC GATGGGGAGG 1260
ACAACACCAT TAGCAGCTGC TTTGACCTGG TGAAGCAGAG CGGGGCCATC ATCCATACAG 1320
TGGCCCTGGG ACCGGCTGCC GCTAAAGAGC TTGAGCAGCT GTCCAAAATG ACAGGAGGCC 1380
TGCAGACATA CTCTTCGGAT CAGGTTCAGA ACAATGGTCT TGTTGATGCT TTCGCAGCAC 1440
TCTCCTCAGG AAATGCGGCG ATCGCTCAGC ACTCCATCCA GCTGGAGAGC AGGGGAGTTA 1500
ATCTCCAGAA TAACCAATGG ATGAATGGCT CAGTGATCGT GGACAGCTCG GTGGGCAAGG 1560
ACACCTTGTT TCTTATCACC TGGACAACGC ATCCTCCTAC AATATTTATC TGGGATCCCA 1620
GCGGAGTGGA ACAAAATGGT TTTATACTAG ACACAACCAC TAAGGTGGCC TACCTCCAAG 1680
TCCCAGGCAC GGCTAAGGTT GGCTTTTGGA AATACAGCAT TCAAGCGAGC TCACAGACTC 1740
TCACCTTGAC TGTCACCTCC CGTGCAGCAA GTGCTACACT GCCTCCTATT ACAGTGACCC 1800
CGGTAGTGAA TAAGAACACA GGGAAATTCC CCAGCCCTGT AACAGTGTAT GCAAGCATTC 1860
GCCAAGGAGC CTCGCCTATT CTCAGGGCCA GCGTCACAGC CTTGATTGAA TCTGTGAATG 1920
GAAAAACAGT AACCCTGGAA TTACTGGATA ACGGAGCAGG TGCCGATGCC ACCAAGAATG 1980
ATGGTGTCTA CTCAAGGTTT TTTACAGCTT TTGATGCAAA TGGTAGATAC AGCGTTAAAA 2040
TATGGGCTCT GGGAGGAGTC ACTTCAGACA GACAGAGAGC AGCACCTCCG AAGAACAGAG 2100

CCATGTACAT AGATGGCTGG ATTGAGGATG GTGAAGTAAG AATGAACCCA CCACGTCCTG 2160
AAACTAGTTA TGTTCAAGAC AAGCAGCTGT GCTTCAGCAG GACATCTTCA GGGGGATCGT 2220
TTGTGGCCAC CAATGTCCCC GCAGCAGCTC CCATTCCTGA CCTCTTTCCA CCCTGTCAAA 2280
TCACTGACCT GAAGGCCAGC ATCCAAGGGC AGAACCTGGT GAATCTGACG TGGACGGCTC 2340
CTGGGGATGA CTACGACCAC GGGAGAGCTT CCAACTACAT CATCCGAATG AGCACCAGTA 2400
TCGTTGATCT CAGGGACCAC TTCAACACCT CACTCCAAGT GAACACTACC GGTCTTATCC 2460
CCAAAGAGGC CAGCTCTGAG GAAATCTTTG AGTTTGAACT GGGAGGCAAC ACTTTTGGAA 2520
ATGGCACAGA TATCTTCATT GCTATCCAGG CTGTGGATAA GTCCAATCTG AAATCAGAAA 2580
TCTCCAACAT TGCACGGGTG TCTGTGTTCA TCCCCGCTCA GGAGCCGCCC ATTCCCGAAG 2640
ACTCAACTCC CCCTTGTCCT GACATCAGCA TCAACAGCAC CATTCCTGGC ATCCACGTGC 2700
TGAAGATAAT GTGGAAGTGG CTAGGGGAAA TGCAGGTGAC ACTAGGTTTG CACTGAATTT 2760
TCAGGCAAGA AATCAACCAG TCATTCCTTT CACTGGAGAA TTTTCTAAAA ATGTACTTTA 2820
GACTTCCTGT AGGGGGCGGT ATA 2843

<210> 23
<211> 486
<212> DNA
<213> Mouse
<400> 23
AGCGGAGTGG AACAAAATGG TTTTATACTA GACACAACCA CTAAGGTGGC CTACCTCCAA 60
GTCCCAGGCA CGGCTAAGGT TGGCTTTTGG AAATACAGCA TTCAAGCGAG CTCACAGACT 120
CTCACCTTGA CTGTCACCTC CCGTGCAGCA AGTGCTACAC TGCCTCCTAT TACAGTGACC 180
CCGGTAGTGA ATAAGAACAC AGGGAAATTC CCCAGCCCTG TAACAGTGTA TGCAAGCATT 240
CGCCAAGGAG CCTCGCCTAT TCTCAGGGCC AGCGTCACAG CCTTGATTGA ATCTGTGAAT 300
GGAAAAACAG TAACCCTGGA ATTACTGGAT AACGGAGCAG GTGCCGATGC CACCAAGAAT 360
GATGGTGTCT ACTCAAGGTT TTTTACAGCT TTTGATGCAA ATGGTAGATA CAGCGTTAAA 420
ATATGGGCTC TGGGAGGAGT CACTTCAGAC AGACAGAGAG CAGCACCTCC GAAGAACAGA 480
GCCATG
486

<210> 24
<211> 21
<212> DNA
<213> Artificial Sequence
<220>
<223> Primer
<400> 24
AATCACAGGG AGATGTACAG C     21

<210> 25
<211> 22
<212> DNA
<213> Artificial Sequence
<220>
<223> Primer
<400> 25
TTTTATAATC AAAAAAAGGA TG     22

<210> 26
<211> 2825
<212> DNA
<213> Human
<400> 26
AATCACAGGG AGATGTACAG CAATGGGGCC ATTTAAGAGT TCTGTGTTCA TCTTGATTCT     60
TCACCTTCTA GAAGGGGCCC TGAGTAATTC ACTCATTCAG CTGAACAACA ATGGCTATGA    120
AGGCATTGTC GTTGCAATCG ACCCCAATGT GCCAGAAGAT GAAACACTCA TTCAACAAAT    180
AAAGGACATG GTGACCCAGG CATCTCTGTA TCTGTTTGAA GCTACAGGAA AGCGATTTTA    240
TTTCAAAAAT GTTGCCATTT TGATTCCTGA AACATGGAAG ACAAAGGCTG ACTATGTGAG    300

ACCAAAACTT GAGACCTACA AAAATGCTGA TGTTCTGGTT GCTGAGTCTA CTCCTCCAGG 360
TAATGATGAA CCCTACACTG AGCAGATGGG CAACTGTGGA GAGAAGGGTG AAAGGATCCA 420
CCTCACTCCT GATTTCATTG CAGGAAAAAA GTTAGCTGAA TATGGACCAC AAGGTAGGGC 480
ATTTGTCCAT GAGTGGGCTC ATCTACGATG GGGAGTATTT GACGAGTACA ATAATGATGA 540
GAAATTCTAC TTATCCAATG GAAGAATACA AGCAGTAAGA TGTTCAGCAG GTATTACTGG 600
TACAAATGTA GTAAAGAAGT GTCAGGGAGG CAGCTGTTAC ACCAAAAGAT GCACATTCAA 660
TAAAGTTACA GGACTCTATG AAAAAGGATG TGAGTTTGTT CTCCAATCCC GCCAGACGGA 720
GAAGGCTTCT ATAATGTTTG CACAACATGT TGATTCTATA GTTGAATTCT GTACAGAACA 780
AAACCACAAC AAAGAAGCTC CAAACAAGCA AAATCAAAAA TGCAATCTCC GAAGCACATG 840
GGAAGTGATC CGTGATTCTG AGGACTTTAA GAAAACCACT CCTATGACAA CACAGCCACC 900
AAATCCCACC TTCTCATTGC TGCAGATTGG ACAAAGAATT GTGTGTTTAG TCCTTGACAA 960
ATCTGGAAGC ATGGCGACTG GTAACCGCCT CAATCGACTG AATCAAGCAG GCCAGCTTTT 1020
CCTGCTGCAG ACAGTTGAGC TGGGGTCCTG GGTTGGGATG GTGACATTTG ACAGTGCTGC 1080
CCATGTACAA AGTGAACTCA TACAGATAAA CAGTGGCAGT GACAGGGACA CACTCGCCAA 1140
AAGATTACCT GCAGCAGCTT CAGGAGGGAC GTCCATCTGC AGCGGGCTTC GATCGGCATT 1200
TACTGTGATT AGGAAGAAAT ATCCAACTGA TGGATCTGAA ATTGTGCTGC TGACGGATGG 1260
GGAAGACAAC ACTATAAGTG GGTGCTTTAA CGAGGTCAAA CAAAGTGGTG CCATCATCCA 1320
CACAGTCGCT TTGGGGCCCT CTGCAGCTCA AGAACTAGAG GAGCTGTCCA AAATGACAGG 1380
AGGTTTACAG ACATATGCTT CAGATCAAGT TCAGAACAAT GGCCTCATTG ATGCTTTTGG 1440
GGCCCTTTCA TCAGGAAATG GAGCTGTCTC TCAGCGCTCC ATCCAGCTTG AGAGTAAGGG 1500
ATTAACCCTC CAGAACAGCC AGTGGATGAA TGGCACAGTG ATCGTGGACA GCACCGTGGG 1560
AAAGGACACT TTGTTTCTTA TCACCTGGAC AACGCAGCCT CCCCAAATCC TTCTCTGGGA 1620
TCCCAGTGGA CAGAAGCAAG GTGGCTTTGT AGTGGACAAA AACACCAAAA TGGCCTACCT 1680
CCAAATCCCA GGCATTGCTA AGGTTGGCAC TTGGAAATAC AGTCTGCAAG CAAGCTCACA 1740
AACCTTGACC CTGACTGTCA CGTCCCGTGC GTCCAATGCT ACCCTGCCTC CAATTACAGT 1800
GACTTCCAAA ACGAACAAGG ACACCAGCAA ATTCCCCAGC CCTCTGGTAG TTTATGCAAA 1860
TATTCGCCAA GGAGCCTCCC CAATTCTCAG GGCCAGTGTC ACAGCCCTGA TTGAATCAGT 1920
GAATGGAAAA ACAGTTACCT TGGAACTACT GGATAATGGA GCAGGTGCTG ATGCTACTAA 1980
GGATGACGGT GTCTACTCAA GGTATTTCAC AACTTATGAC ACGAATGGTA GATACAGTGT 2040

AAAAGTGCGG GCTCTGGGAG GAGTTAACGC AGCCAGACGG AGAGTGATAC CCCAGCAGAG 2100
TGGAGCACTG TACATACCTG GCTGGATTGA GAATGATGAA ATACAATGGA ATCCACCAAG 2160
ACCTGAAATT AATAAGGATG ATGTTCAACA CAAGCAAGTG TGTTTCAGCA GAACATCCTC 2220
GGGAGGCTCA TTTGTGGCTT CTGATGTCCC AAATGCTCCC ATACCTGATC TCTTCCCACC 2280
TGGCCAAATC ACCGACCTGA AGGCGGAAAT TCACGGGGGC AGTCTCATTA ATCTGACTTG 2340
GACAGCTCCT GGGGATGATT ATGACCATGG AACAGCTCAC AAGTATATCA TTCGAATAAG 2400
TACAAGTATT CTTGATCTCA GAGACAAGTT CAATGAATCT CTTCAAGTGA ATACTACTGC 2460
TCTCATCCCA AAGGAAGCCA ACTCTGAGGA AGTCTTTTTG TTTAAACCAG AAAACATTAC 2520
TTTTGAAAAT GGCACAGATC TTTTCATTGC TATTCAGGCT GTTGATAAGG TCGATCTGAA 2580
ATCAGAAATA TCCAACATTG CACGAGTATC TTTGTTTATT CCTCCACAGA CTCCGCCAGA 2640
GACACCTAGT CCTGATGAAA CGTCTGCTCC TTGTCCTAAT ATTCATATCA ACAGCACCAT 2700
TCCTGGCATT CACATTTTAA AAATTATGTG GAAGTGGATA GGAGAACTGC AGCTGTCAAT 2760
AGCCTAGGGC TGAATTTTTG TCAGATAAAT AAAATAAATC ATTCATCCTT TTTTTGATTA 2820
TAAAA
2825

<210> 27
<211> 20
<212> DNA
<213> Artificial Sequence
<220>
<223> Primer
<400> 27
AAGGTAAGGC ATTTGTCCAT 20

<210> 28
<211> 20
<212> DNA
<213> Artificial Sequence

<220>
<223> Primer
<400> 28
ATGGACAAAT GCCTTACCTT 20

<210> 29
<211> 20
<212> DNA
<213> Artificial Sequence
<220>
<223> Primer
<400> 29
GAAGCATGGC GACTGGTAAC 20

<210> 30
<211> 20
<212> DNA
<213> Artificial Sequence
<220>
<223> Primer
<400> 30
GTTACCAGTC GCCATGCTTC 20

<210> 31
<211> 20
<212> DNA
<213> Artificial Sequence
<220>
<223> Primer

<400> 31
TCCATCCAGC TTGAGAGTAA 20

<210> 32
<211> 20
<212> DNA
<213> Artificial Sequence
<220>
<223> Primer
<400> 32
TTACTCTCAA GCTGGATGGA 20

<210> 33
<211> 20
<212> DNA
<213> Artificial Sequence
<220>
<223> Primer
<400> 33
TGATGCTACT AAGGATGACG 20

<210> 34
<211> 20
<212> DNA
<213> Artificial Sequence
<220>
<223> Primer
<400> 34
CGTCATCCTT AGTAGCATCA 20

<210> 35
<211> 20
<212> DNA
<213> Artificial Sequence
<220>
<223> Primer
<400> 35
AGGAAGCCAA CTCTGAGGAA 20

<210> 36
<211> 20
<212> DNA
<213> Artificial Sequence
<220>
<223> Primer
<400> 36
TTCCTCAGAG TTGGCTTCCT 20

<210> 37
<211> 21
<212> DNA
<213> Artificial Sequence
<220>
<223> Primer
<400> 37
CTGATGTTCT GGTTGCTGAG T 21

<210> 38

<211> 20
<212> DNA
<213> Artificial Sequence
<220>
<223> Primer
<400> 38
TTGGAGCTTC TTTGTTGTGG     20

<210> 39
<211> 20
<212> DNA
<213> Artificial Sequence
<220>
<223> Anti-sense
<400> 39
GACTCTTCAA AGATTCCATC     20

<210> 40
<211> 20
<212> DNA
<213> Artificial Sequence
<220>
<223> Anti-sense
<400> 40
GAGTCATCTT AGTTTCGTTC     20